# EUROPEAN PATENT SPECIFICATION

(11) **EP 3 796 933 B1**
(45) Date of publication and mention of the grant of the patent: **23.09.2026**
(21) Application number: 19803740.0
(22) Date of filing: 17.05.2019
(51) Int. Cl.: C12N 15/11, A61K 39/395, A61K 48/00, A61P 35/00

(54) **METHODS FOR TREATING, PREVENTING AND DETECTING THE PROGNOSIS OF COLORECTAL CANCER**
VERFAHREN ZUR BEHANDLUNG, VORBEUGUNG UND DETEKTION DER PROGNOSE VON KOLOREKTALKREBS
MÉTHODES DE TRAITEMENT, PRÉVENTION ET DÉTECTION DU PRONOSTIC DU CANCER COLORECTAL

(30) Priority: 17.05.2018 US 201862672989 P; 24.01.2019 US 201962796349 P
(43) Date of publication of application: 31.03.2021
(73) Proprietor: Han, Yiping, Paramus, NJ 07652 (US)
(72) Inventor: HAN, Yiping, New York, NY 10463 (US); DALERBA, Piero, New York, NY 10027 (US)
(74) Representative: Maiwald GmbH
(86) International application number: PCT/US2019/032855
(87) International publication number: WO 2019/222618

(56) References cited:
- WO-A1-2009/114836
- WO-A1-2013/052480
- WO-A1-2017/119484
- WO-A2-2010/036924
- US-A1- 2011 287 958
- US-A1- 2012 251 453
- US-A1- 2015 086 553
- EMIKO TAKESHITA ET AL.: "Alternative treatments for prophylaxis of colorectal cancer in familial adenomatous polyposis", JOURNAL OF THE ANUS, RECTUM AND COLON, vol. 1, no. 3, 1 January 2017 (2017-01-01), pages 74 - 77, XP055947169, Retrieved from the Internet <URL:https://www.ncbi.nlm.nih.gov/pmc/articles/PMC6768673/pdf/2432-3853-1-0074.pdf> [retrieved on 20220728]
- HISASHI ONOZAWA ET AL.: "Annexin A1 is involved in resistance to 5-FU in colon cancer cells", ONCOLOGY REPORTS, vol. 37, no. 1, 8 November 2016 (2016-11-08), pages 235 - 240, XP055655980, ISSN: 1021-335X, DOI: 10.3892/or.2016.5234
- ONOZAWA ET AL.: "Annexin A1 is involved in resistance to 5-FU in colon cancer cells", ONCOLOGY REPORTS, vol. 37, no. 1, 8 November 2016 (2016-11-08), pages 235 - 240, XP055655980
- GASPAR ET AL.: "Conserved Mechanisms in Adenomatous Polyposis Coli (APC)-Driven Tumorigenesis", THE AMERICAN JOURNAL OF PATHOLOGY, vol. 172, no. 5, May 2008 (2008-05-01), pages 1363 - 1380, XP055655981

## Description

### FIELD OF THE INVENTION

The present invention relates to agents that inhibit or block Annexin A1 for use in treating cancer or preventing cancer in a subject with familial adenomatous polyposis.

### BACKGROUND OF THE INVENTION

Colorectal cancer (CRC) is the second leading cause of cancer death in the United States, affecting 1 in 20 individuals (ACS 2012). CRC has long been recognized to result from host mutations that accumulate over time, developing from precancerous adenomatous polyps into adenocarcinoma over approximately ten years (Vogelstein and Kinzler 1993). Advancements in microbial detection technology and human microbiome research have revolutionized our understanding of a wide spectrum of diseases, including CRC (Dulal and Keku 2014; Keku *et al.* 2015). However, there is still a need for more effective treatments for CRC, and as well as other solid tumors.

Recent studies identified *Fusobacterium nucleatum (F. nucleatum* or *Fn*), a Gram-negative oral commensal bacterium, as a significant factor in colorectal cancer (CRC) that is often associated with poor prognosis (Bullman *et al.* 2017; Yu *et al.* 2017). *Fn* stimulates colorectal tumorigenesis through binding of its FadA adhesin to E-cadherin, which then activates Wnt/β-catenin signaling (Rubinstein *et al.* 2013). E-cadherin is expressed ubiquitously, and it was not known why binding of FadA to E-cadherin on CRC cells differs from binding to other cells.

*Fn* has been detected in approximately 10-90% CRC tissues (Tahara *et al.* 2014; Mima *et al.* 2016; Yu *et al.* 2016), with higher prevalence in the proximal colon than distal colon (Mima *et al.* 2016a; Yu *et al.* 2016). *Fn* is often associated with advanced disease, chemo-resistance, metastasis, and poor prognosis (Bullman *et al.* 2017; Yu *et al.* 2017). A few studies have supported a causal role of *Fn* in CRC (Yu *et al.* 2016; Rubinstein *et al.* 2013). It was reported that *Fn* promotes CRC growth through its unique FadA adhesin, which binds to E-cadherin (CDH1) and activates Wnt/β-catenin signaling (Rubinstein *et al.* 2013). Binding of FadA to E-cadherin requires both the intact pre-FadA consisting of 129 amino-acid residues and the mFadA of 111 amino-acid residues without the signal peptide. Together they form the FadAc complex (Rubinstein *et al.* 2013; Xu *et al.* 2007). However, FadA did not promote growth of non-cancerous HEK293 cells even when E-cadherin was present (Rubinstein *et al.* 2013), leading to the question of whether *F. nucleatum-*induced growth is specific to CRC and if so by what mechanism.

Familial adenomatous polyposis (FAP) is an inherited colorectal cancer syndrome and accounts for 1 percent of all cases of colorectal cancer. The "F" stands for familial, meaning it runs in families; "A" stands for adenomatous, the type of polyps detected in the colon and small intestine that can turn into cancer; and "P" stands for polyposis, or the condition of having many colon polyps. The gene for FAP is on the long arm of chromosome 5 and is called the APC gene.

Patients with FAP develop hundreds to thousands of colon polyps, usually starting in the teens. All patients will develop colorectal cancer from the colon polyps usually by age 40. Patients with FAP must have the colon, and sometimes the rectum, removed to prevent colon cancer. The only current pharmacological treatment, sulindac and celecoxib, have not been successful, due to side effects and lack of efficacy. Thus there is a need for better prevention and treatment options for patients with FAP.

US20110287958A1 describes methods of determining a colorectal tumor stage in a patient by gene expression analysis. The method comprises assaying the level of at least one RNA transcript, or its expression product, which is correlated to colorectal tumor stage and may be useful for determining whether a patient has stage II or stage III colorectal cancer.

WO2013/052480A1 describes methods and compositions for the prognosis and classification of cancer, especially colon cancer. For example, methods for colon cancer prognosis using expression analysis of selected biomarkers are described. It is described that a risk score may be developed to provide a cancer prognosis.

WO2010036924A2 describes methods for detecting a more aggressive form of a colon adenocarcinoma in a subject, thereby predicting the prognosis of the subject. The methods generally include determining an inflammatory gene expression signature in the colon adenocarcinoma and/or the adjacent non-cancerous tissue. The inflammatory genes may include PRGl, ANXAl, IL-17a, IL-23a FOXP3, HLA-DRA, IL-IO, CD68 and IL- 12a. The method may further include detecting expression of microRNA-21 (miR-21) in the colon adenocarcinoma. Altered expression of one or more of the inflammatory genes or miR-21 indicates the prognosis of the subject. Also described are arrays consisting essentially of probes specific for PRGl, ANXAl, IL-17a, IL- 23a, FOXP3, HLA-DRA, IL-IO, CD68, IL-12a and miR-21.

US20150086553A1 describes a polypeptide binding to Annexin A1, an antagonist against Annexin A1 including the polypeptide, an anti-Annexin A1 antibody including the polypeptide or an antigen-binding fragment thereof, and methods of preventing, treating and/or diagnosing a disease, including administering the antagonist and/or the antibody or an antigen-binding fragment thereof to a subject.

WO2009114836A1 describes gene sets which are useful in assessing prognosis and/or predicting the response of cancer, e.g. colorectal cancer to chemotherapy. Also described is a clinically validated cancer test, e.g. colorectal test, for assessment of prognosis and/or prediction of patient response to chemotherapy, using expression analysis. It is described that the use of archived paraffin embedded biopsy material for assay of all markers in the relevant gene sets is accommodated for, and therefore is compatible with the most widely available type of biopsy material.

Hisashi Onozawa et al. (Annexin A1 is involved in resistance to 5-FU in colon cancer cells, Oncology Reports, 2026, 37, 1, p. 235-240) describes that Annexin A1 is involved in chemoresistance in colon cancer cells.

Emiko Takeshita et al. (Alternative treatments for prophylaxis of colorectal cancer in familial adenomatous polyposis, Journal of the Anus, Rectum and Colon, 2017, 1, 3, p. 74-77) describes treatments for prophylaxis of colorectal cancer in familial adenomatous polyposis.

### SUMMARY OF THE INVENTION

The present invention is based upon the novel findings set forth herein that *Fusobacterium nucleatum* (*F. nucleatum* or *Fn*), a Gram-negative oral anaerobe, is a significant contributor to colorectal cancer. *F*. *nucleatum* stimulates the growth of colorectal cancer cells without affecting the pre-cancerous adenoma cells. Annexin A1, a previously unrecognized modulator of Wnt/β-catenin signaling, is a key component through which *F*. *nucleatum* exerts its stimulatory effect. It is specifically expressed in proliferating colorectal cancer cells and involved in activation of cyclin D1. The FadA adhesin from *F. nucleatum* up-regulates Annexin A1 expression through E-cadherin. A positive feedback loop between FadA and Annexin A1 was identified in the cancerous cells and absent in the non-cancerous cells. A "two-hit" model in colorectal carcinogenesis, with somatic mutation(s) as the first hit, and *F. nucleatum* as the second hit exacerbating cancer progression after benign cells become cancerous, is hypothesized.

Embodiments of the present invention are based upon Annexin A1 (*ANXA1*), selectively expressed in proliferating CRC cells and specifically induced by *Fn,* as a suitable therapeutic target. It is disclosed herein that that *Fn* induces chemo-resistance by activating Annexin A1, and by blocking Annexin A1, thus chemoresistance may be overcome. The instant invention elucidates a novel role for Annexin A1 in modulating Wnt/β-catenin signaling. Given the broad implication of Wnt/β-catenin in cancer, and increasing numbers of reports of *Fn* in different types of cancer, including malignant solid tumors including melanoma (*e.g.,* metastatic malignant melanoma), renal cancer (*e.g.* clear cell carcinoma), prostate cancer (*e.g.* hormone refractory prostate adenocarcinoma), pancreatic adenocarcinoma, breast cancer, colon cancer (CRC), lung cancer (*e.g*. non-small cell lung cancer), esophageal cancer, squamous cell carcinoma of the head and neck, liver cancer, ovarian cancer, cervical cancer, thyroid cancer, glioblastoma, glioma, and leukemia, Annexin A1 is a novel therapeutic target for reducing cancer cell proliferation and cancer treatment.

The present invention is directed to an agent that inhibits or blocks Annexin A1 for use in treating cancer or preventing cancer in a subject with familial adenomatous polyposis, wherein the agent that inhibits or blocks Annexin A1 is selected from the group consisting of an *ANXA1*-specific siRNA, an *ANXA1*-specific miRNA, DNA encoding an *ANXA1*-specific siRNA, DNA encoding an *ANXA1*-specific miRNA, and an anti-Annexin A1 antibody, minibody, Fab or fragment, camelid, or nanobody.

In some embodiments, the cancer is selected from the group consisting of melanoma, renal cancer, prostate cancer, pancreatic adenocarcinoma, breast cancer, colon or colorectal cancer (CRC), lung cancer, esophageal cancer, squamous cell carcinoma of the head and neck, liver cancer, ovarian cancer, cervical cancer, thyroid cancer, glioblastoma, glioma, and leukemia.

In some embodiments, the agent further comprises a vector, liposome, nanocapsule, nanoparticle, microparticle, microsphere, lipid particle, or vesicle.

In some embodiments, the agent further comprises a pharmaceutically acceptable, diluent, carrier or adjuvant.

In some embodiments, the agent is for use in combination with a therapeutic agent selected from the group consisting of a chemotherapeutic agent, a targeted chemotherapeutic agent, an immunotherapeutic agent and combinations thereof.

In some embodiments, the chemotherapeutic agent is chosen from the group consisting of microtubule-targeting moietys (MTAs), DNA damaging agents, alkylating agents, antimetabolites, spindle poison plant alkaloids, cytotoxic/antitumor antibiotics, topoisomerase inhibitors, antibodies, photosensitizers, and kinase inhibitors.

In some embodiments, the targeted chemotherapeutic agent is chosen from the group consisting of denosumab, romidepsin, ofatumumab, pazopanib, everlimus, nilotinib, temisirolimus, lapatinib, sunitinib, dasatinib, vorinostat, erlotinib, bevacizumab, cetuximab, bortezomib, gefitinib, ibritumomab, alemtuzumab, imatinib, gentuzumab, denileukin difitox, trastumab, rituximab, ramucirumab, ziv-aflibercept, panitumumab, and regorafenib.

In some embodiments, the immunotherapeutic agent is selected from the group consisting of pembrolizumab, nivolumab, and ipilimumab.

In some embodiments, the the cancer is colorectal cancer and the chemotherapeutic agent is selected from the group consisting of 5-fluorouracil, capecitabine, irinotecan, oxaliplatin, and a combination or trifluridine and tipiracil, the targeted chemotherapeutic agent is chosen from the group consisting of bevacizumab, ramucirumab, ziv-aflibercept, cetuximab, panitumumab, and regorafenib, and the immunotherapeutic agent is selected from the group consisting of pembrolizumab, nivolumab, and ipilimumab.

In some embodiments, the agent reduces chemo-resistance in the subject being treated for cancer with a chemotherapeutic agent.

### BRIEF DESCRIPTION OF THE DRAWINGS

For the purpose of illustrating the invention, there are depicted in drawings certain embodiments of the invention. However, the invention is not limited to the precise arrangements and instrumentalities of the embodiments depicted in the drawings.

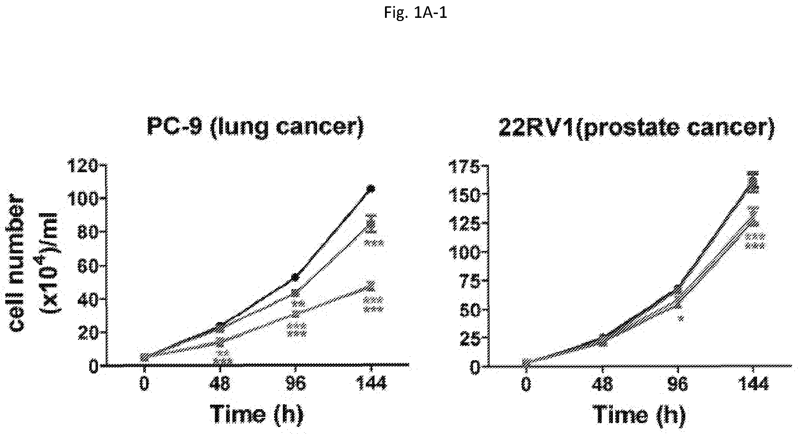

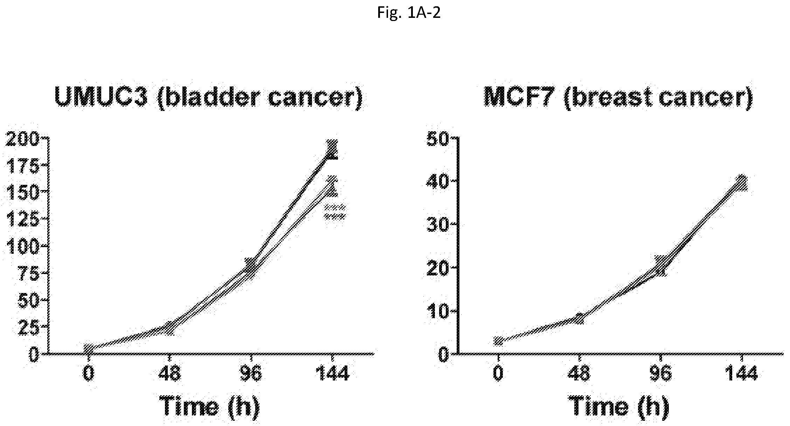

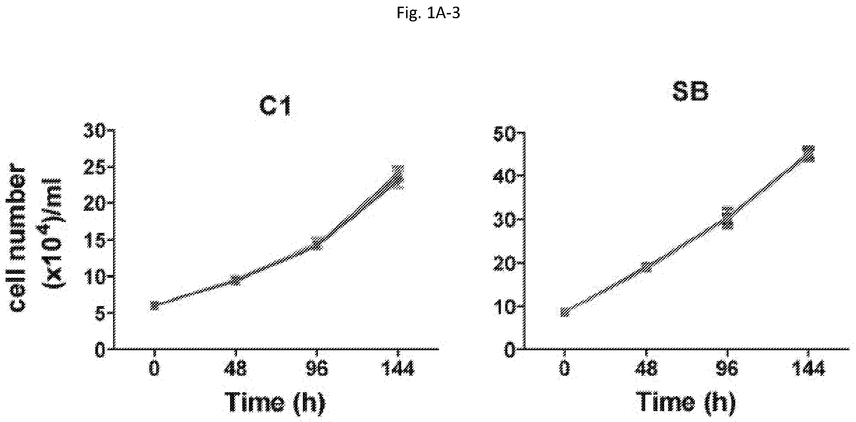

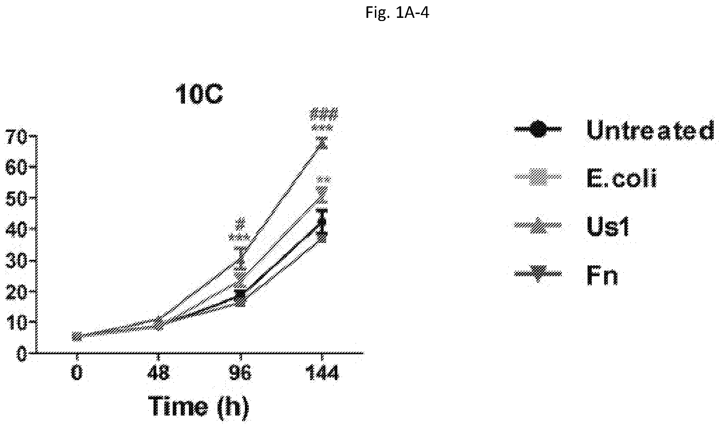

**Figs. 1A-1K** show that *F. nucleatum* preferentially binds, invades and stimulates the growth of cancerous colorectal cells via Annexin A1, and Annexin A1 is selectively expressed in proliferating cancerous colorectal cells and is a novel CRC growth factor, and that *ANXA1*-specific siRNA inhibits tumor growth.
Fig. 1A show the results of a cell proliferation assay of lung cancer cells PC-9, prostate cancer cells 22RV1, bladder cancer cells UMUC3, breast cancer cells MCF-7, colonic adenoma-derived non-cancerous cells AA/C1 ("C1") and AA/C1/SB ("SB"), or cancerous cells AA/C1/SB/10C ("10C") following incubation with wild-type *F*. *nucleatum* 12230 (*Fn*), the *fadA*-deletion mutant US1 (US1), or *E. coli* DH5α (*E. coli*) at multiplicity of infection (MOI) of 1000: 1. Cells were counted at the indicated times. Cell numbers are mean values ± SEM. The experiment was performed in triplicates and repeated three times. **p<0.01, ***p<0.001, compared to untreated controls; #p<0.05, ### p<0.001, compared to US1-treated cells (two-way ANOVA).

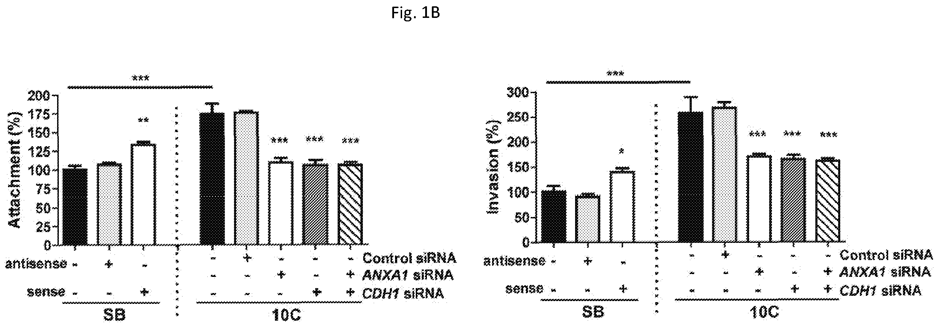

Fig. 1B shows the results of the attachment (left panel) and invasion (right panel) of wild-type *F*. *nucleatum* 12230 (MOI=50:1) to the non-tumorigenic SB cells, either untreated or transfected with antisense or sense *ANXA1,* and to the tumorigenic 10C cells, either untreated or transfected with control or *ANXA1-* or *CDH1*-specific siRNA or both. *Fn* attachment and invasion to the untreated SB cells were designated as 100%, respectively; all other values were expressed as relative to those obtained with untreated SB. Data are mean values ± SEM. *p<0.05, ** p<0.01 and *** p<0.001 (one-way ANOVA).

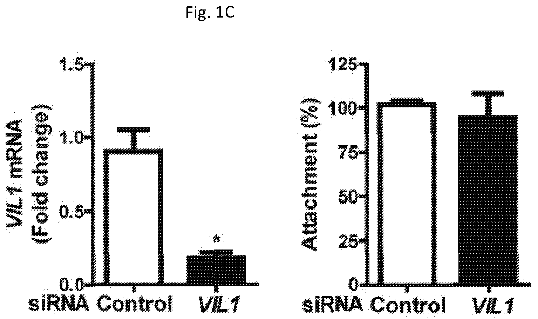

Fig. 1C are graphs of the results of qPCR analysis of Villin1 (*VIL1*) mRNA levels in 10C cells treated with control siRNA or *VIL1*-specific siRNA, demonstrating knocking down of Villin1 (left panel) and the attachment of *F. nucleatum* 12230 to 10C cells treated with control siRNA or *VIL1*-specific siRNA (right panel). Data are mean values ± SD. The experiment was performed in triplicates and repeated twice. *p<0.05 (student's t-test).

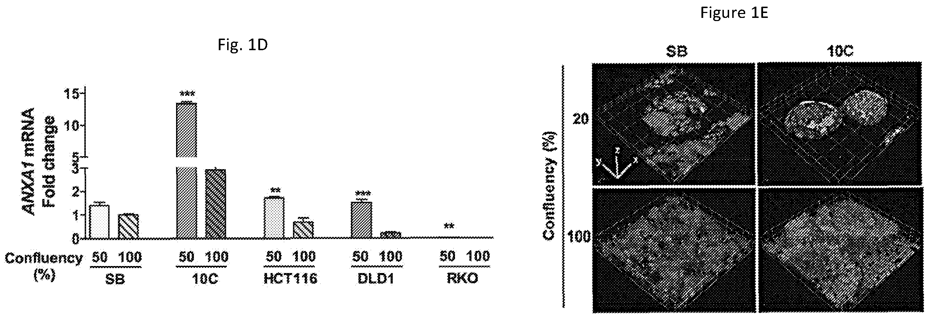

Fig. 1D is a graph of the results of real-time qPCR analysis of *ANXA1* expression using mRNA extracted from the noncancerous SB, cancerous 10C, and human CRC cell lines HCT116, DLD1 and RKO, each grown to 50% or 100% confluency. All results were normalized to the *ANXA1* mRNA levels in SB cells of 100% confluency, which was designated as 1. Data are mean values ± SEM. The experiment was performed in triplicates and repeated twice. **p<0.01 and ***p<0.001 (student's t-test).
Fig. 1E are representative images from confocal microscopy analysis of SB and 10C cells grown to 20% (top panels) or 100% (bottom panels) confluency followed by immunofluorescent staining of Annexin A1 (green) and DAPI staining of the nuclei (blue). A series of 20-50 consecutive images in the z axis were stacked together to generate the 3-D figure at 400x magnification. Annexin A1 was most abundantly expressed on the outer layer of 20% confluent 10C (pointed by arrows), compared to 100% confluent 10C, or SB of either confluency. Scale bars, x = 1 µm, y = 1 µm, z = 1.6 µm. The experiment was repeated at least twice.

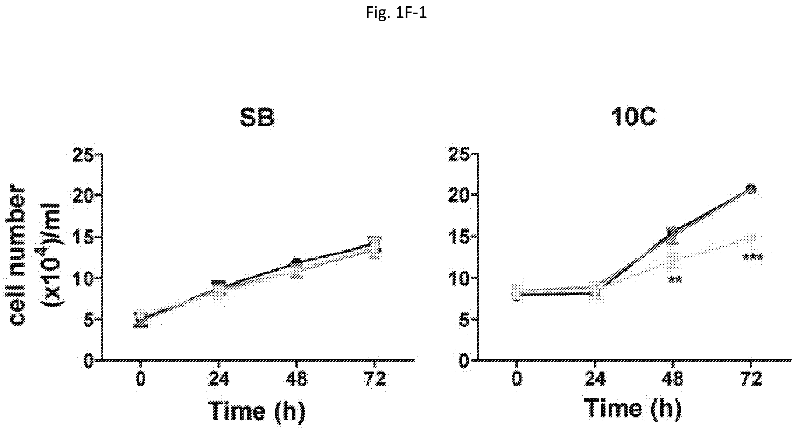

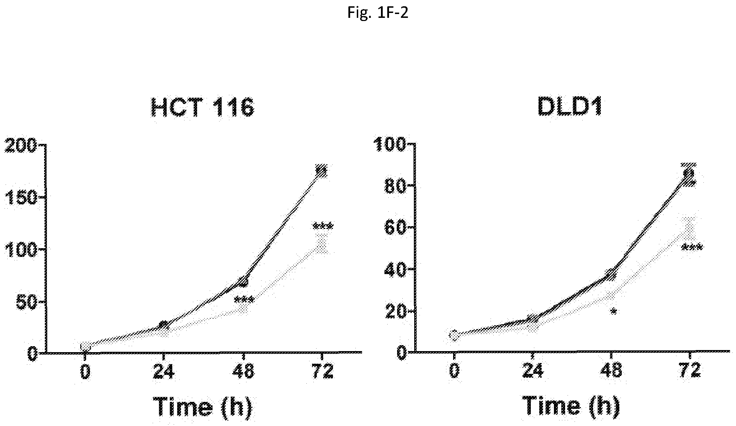

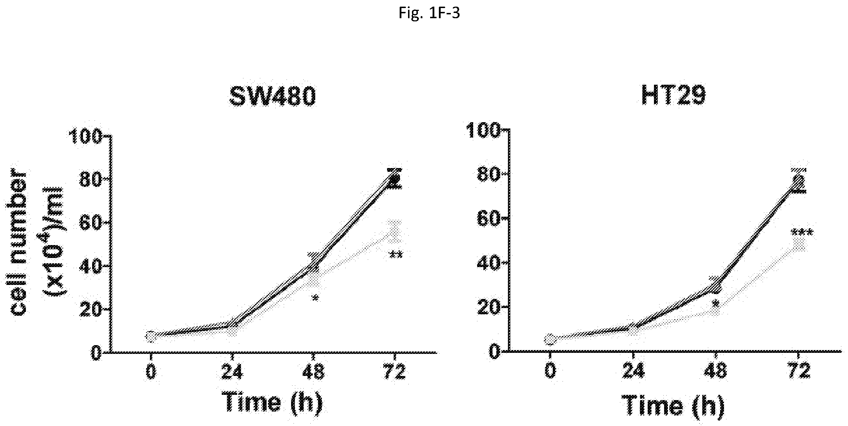

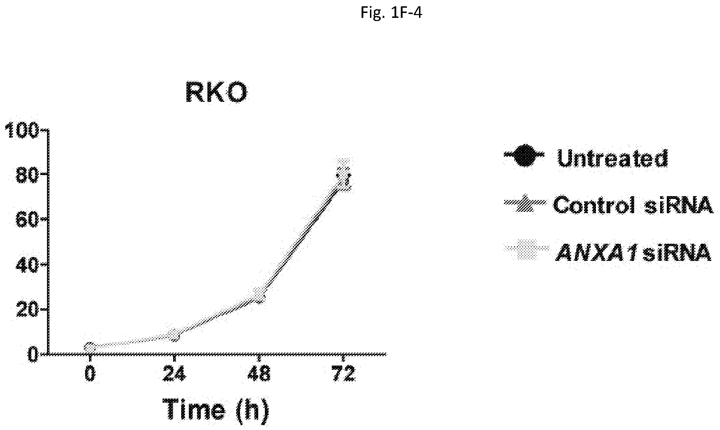

Fig. 1F is a graph of the results of a cell proliferation assay of adenoma-derived non-tumorigenic SB and tumorigenic 10C and human CRC cell lines HCT116, DLD1, SW480, HT29 and RKO either untreated (black lines with circles) or following treatment with control siRNA (gray lines with triangles) or *ANXA1*-specific siRNA (gray lines with squares). Data are mean values ± SEM. The experiment was performed in triplicates and repeated three times. *p<0.05, ** p<0.01 and *** p<0.001, compared to the untreated cells (two-way ANOVA).

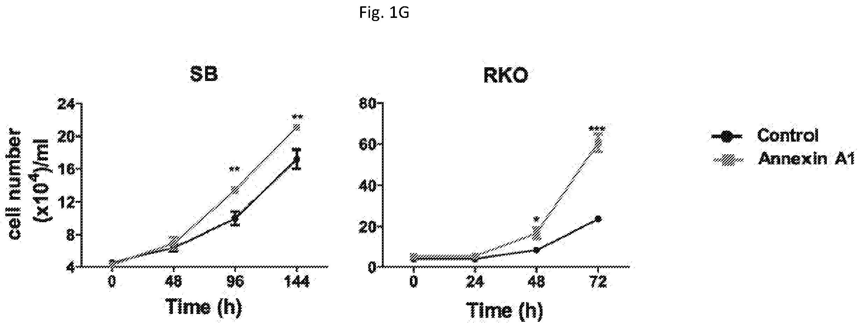

Fig. 1G are graphs of the results of a cell proliferation assay of SB (left panel) and RKO (right panel) cells transfected with *ANXA1* (gray line with squares), as compared to the control cells (black lines with circles). Data are mean values ± SEM. The experiment was performed in triplicates and repeated three times. *p<0.05, **p<0.01 and ***p<0.001 (two-way ANOVA).

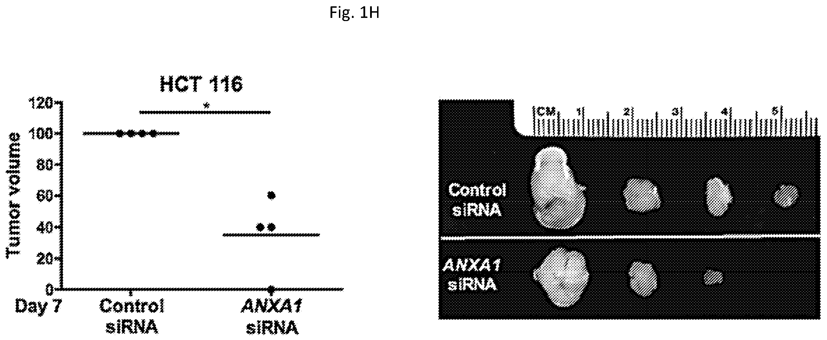

Fig. 1H are graphs and images of xenografted tumor growth in nude mice following subcutaneous and bilateral inoculation of HCT116 cells transfected with control siRNA (left side) or *ANXA1*-specific siRNA (right side). The tumor volumes were measured after 7 days post injection (left panel, n=4). For each mouse, the tumor resulting from *ANXA1* siRNA-treated cells was normalized to that arising from control siRNA-treated cells which was designated to 100%. The line represents the average. *p<0.05 (unpaired t-test). Representative tumors are shown on the right panel top, tumors arising from control siRNA treated cells; bottom, tumors arising from *ANXA1*-specific siRNA treated cells.

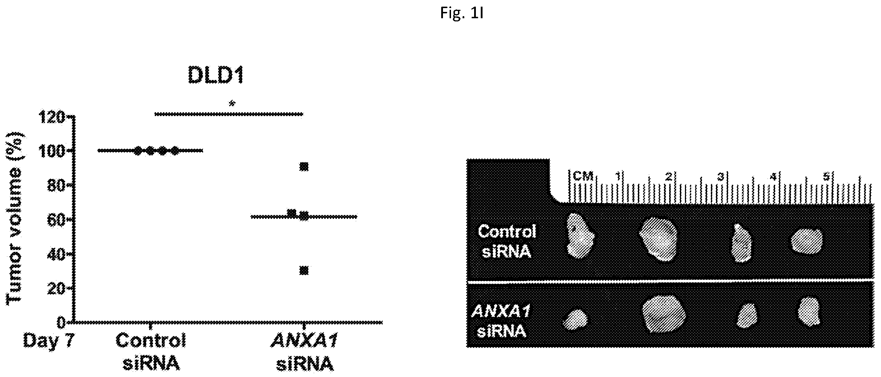

Fig. 1I are graphs and images of xenografted tumor growth in nude mice following subcutaneous and bilateral inoculation of DLD1 cells transfected with control siRNA (left side) or *ANXA1*-specific siRNA (right side). The tumor volumes were measured after 7 days post injection (left panel, n=3). For each mouse, the tumor resulting from *ANXA1* siRNA-treated cells was normalized to that arising from control siRNA-treated cells which was designated to 100%. The line represents the average. *p<0.05 (unpaired t-test). Representative tumors are shown on the right panel top, tumors arising from control siRNA treated cells; bottom, tumors arising from *ANXA1*-specific siRNA treated cells.
Fig. 1J shows IL 1β, Nfkb2, Rantes, CCL20 and cyclin D1 expression measured by real-time qPCR in MCF-7, AA/C1, AA/C1/SB (SB) and AA/C1/SB/10C (10C) cells incubated with wild-type *Fn 12230* for 3 hours then RNA was extracted. Data are mean values ± SD. *p<0.05, ** p<0.01 and *** p<0.001 compared to untreated cells.

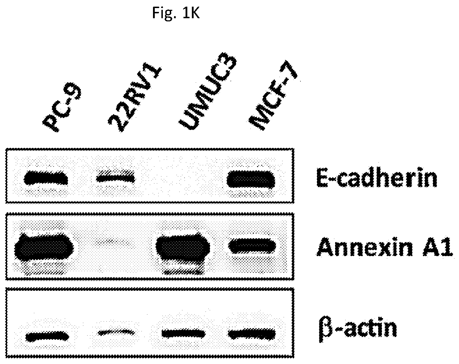

Fig. 1K shows Western blot analysis of E-cadherin and Annexin A1 expression in PC-9 lung cancer cells, 22RV1 prostate cancer cells, UMUC3 bladder cancer cells and MCF-7 breast cancer cells. β-actin was included as an internal control.

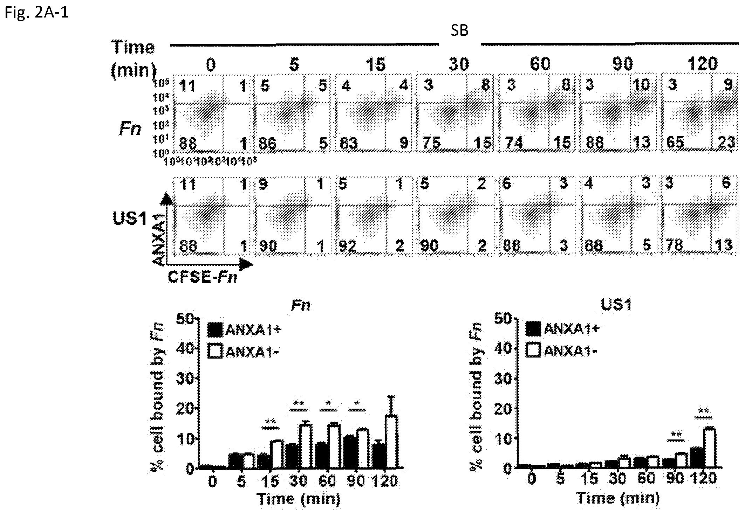

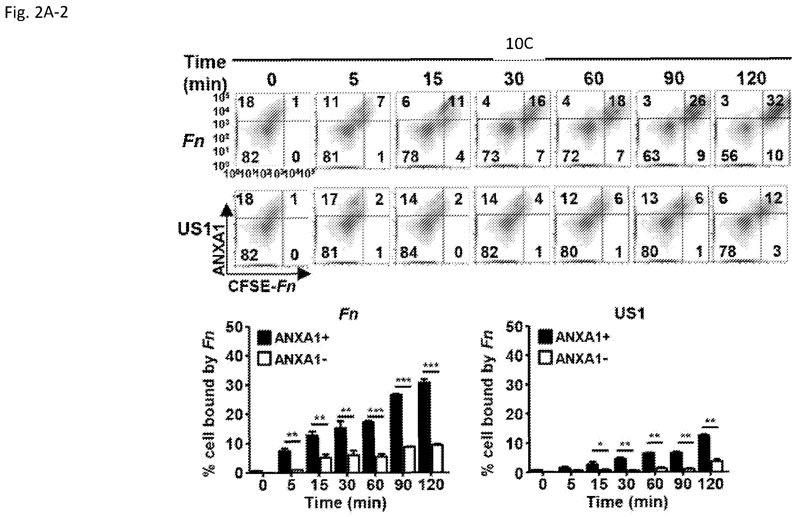

**Figs. 2A-2F** show that *F. nucleatum* selectively binds to Annexin A1-expressing cells and induces further Annexin A1 expression via FadA.
Fig. 2A are representative images and graphs of flow cytometry analysis of SB and 10C cells following incubation with CFSE-labeled *F*. *nucleatum* 12230 (*Fn*) or its *fadA-*deletion mutant US1 (US1) for the indicated time and subsequent immuno-staining of Annexin A1. Shown on the top panels are the density plots. x-axis, Annexin A1; y-axis, CFSE-labeled *Fn* or US1. Shown on the bottom panels are the geometric means of Annexin A1-positive staining (lines, scale on the right) and the percentages of Annexin A1-positive (solid bars) or negative (clear bars) cells bound by *Fn* or US1 out of the total number of cells analyzed (scale on the left). Data are mean values ± SD. *p<0.05, *** p<0.001.

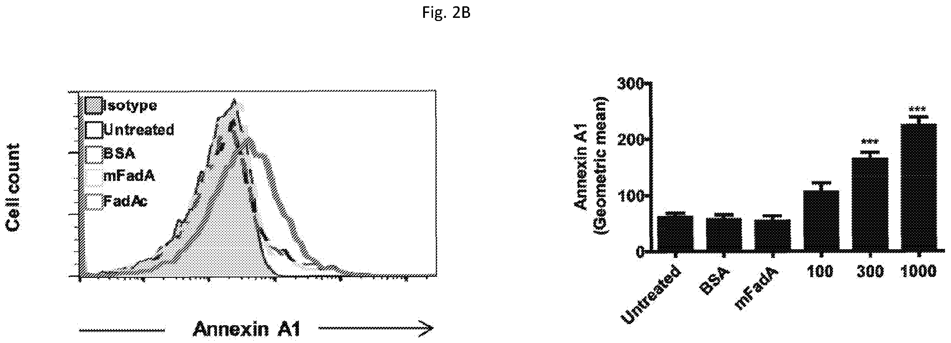

Fig. 2B are graphs of the results of flow cytometry analysis of Annexin A1 expression in 10C cells either untreated or incubated with BSA (1000 µg/ml), or mFadA (1000 µg/ml), or FadAc (100, 300, or 1000 µg/ml). Data are mean values ± SD. ** p<0.01.

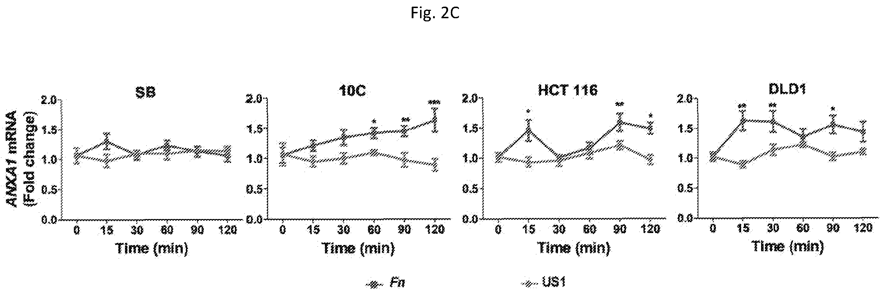

Fig. 2C are graphs of the results of real-time qPCR analysis of *ANXA1* mRNA levels in SB, 10C, HCT116 and DLD1 cells treated with wild-type *F*. *nucleatum* 12230 (*Fn*) (dark lines with squares) or *fad*A-deletion mutant US1 (light line with circles) for the indicated time periods. The results were normalized to those obtained from untreated cells and were the mean of three independent experiments each performed in triplicates and *p<0.05, **p<0.01 and ***p<0.001 (two-way ANOVA).

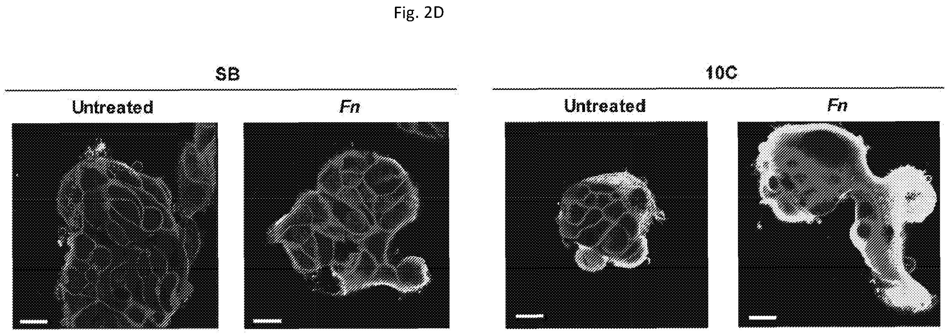

Fig. 2D are representative images of confocal microscopy analysis of SB and 10C cells either untreated or following incubation with *F*. *nucleatum* 12230 (*Fn*) for 1 hour at MOI of 5:1. Annexin A1 was stained green (appearing white in the image) while E-cadherin blue (appearing gray in the image). Imagines were 800x magnification. Note the enhanced expression of Annexin A1 in 10C compared to SB and its location on the outer rim of the cell mass. The experiment was repeated three times. Scale bar, 250 nm.

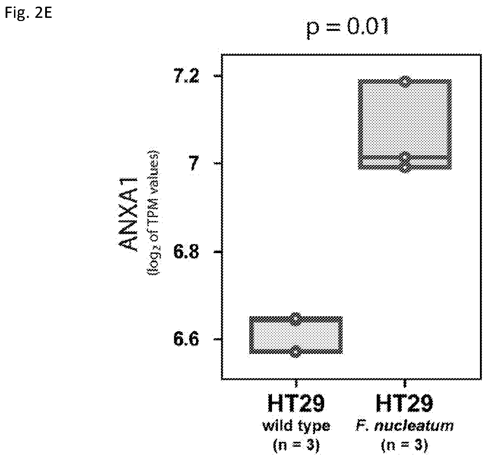

Fig 2E shows the statistical analysis of associations between exposure to *F*. *nucleatum* and upregulation of *ANXA1* mRNA expression levels in colon cancer cell HT29. The *ANXA1* mRNA levels in HT29 cells were analyzed in an RNA-sequencing (RNA-seq) dataset publicly available from the NCBI-GEO online repository (GSE90944) and containing global gene-expression measurements from HT29 cells, both at baseline and following incubation with *F*. *nucleatum* 25586 in triplicates (Yu *et al.* 2017). The distribution of *ANXA1* mRNA expression levels in the two sample groups (baseline vs. infected) was visualized using box-plots, using the log2 of their TPM (transcripts per million) expression values as a metric. Differences in mean log2 TPM values between HT29 cells at baseline (n=3) and following incubation with *F. nucleatum* (n=3) were tested for statistical significance using a two-tailed t-test for continuous variables. The analysis revealed that HT29 cells exposed to *F. nucleatum* were characterized by increased levels of *ANXA1* mRNA expression, as compared to HT29 cells at baseline (p = 0.01).

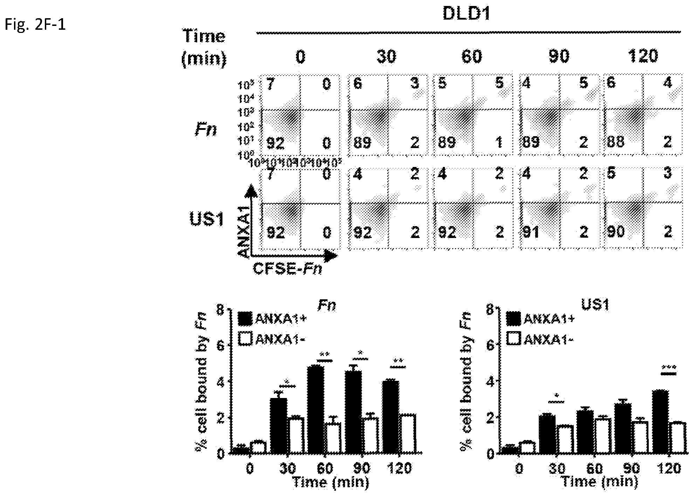

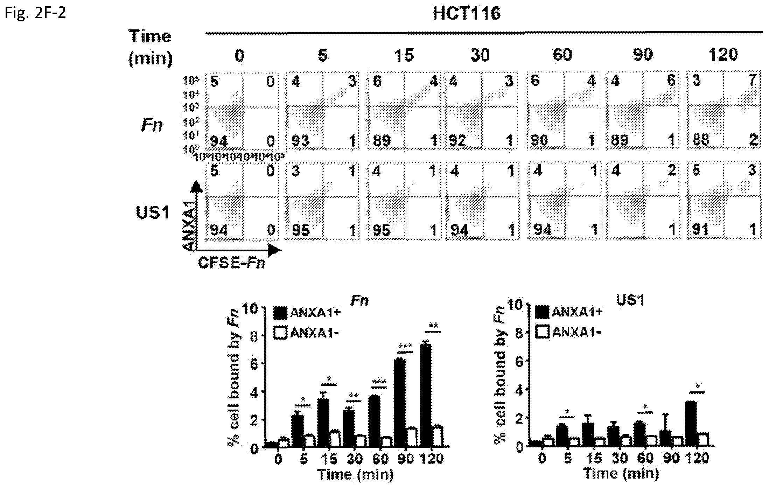

Fig. 2F are representative images and graphs of flow cytometry analysis of DLD1 and HCT116 cells following incubation with CFSE-labeled *F. nucleatum* 12230 (Fn) or its*fad*A-deletion mutant US1 (US1) for the indicated time and subsequent immuno-staining of Annexin A1. Shown on the top panels are the density plots. x-axis, Annexin A1; y-axis, CFSE-labeled *Fn* or US1. Shown on the bottom panels are the geometric means of Annexin A1-positive staining (blue lines, scale on the right) and the percentages of Annexin A1-positive (solid bars) or negative (clear bars) cells bound by Fn or US1 out of the total number of cells analyzed (scale on the left). Data are mean values ± SD. *p<0.05, *** p<0.001.

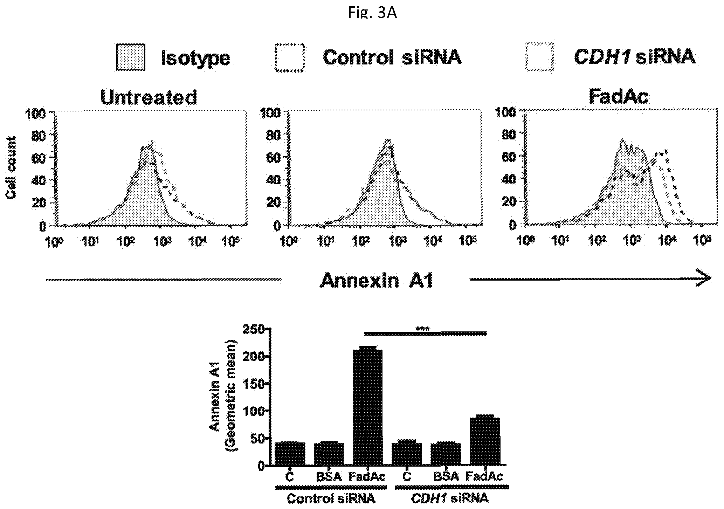

**Figs. 3A****-3L** show FadA, E-cadherin (CDH1), Annexin A1 and β-catenin form a complex in cancerous cells.
Fig. 3A are graphs of the results of flow cytometry analysis of Annexin A1 in 10C cells transfected with control siRNA (dotted black line) or *CDH1*-specific siRNA (dotted red line) followed by no treatment, or incubation with BSA (1000 µg/ml) or FadAc (1000 µg/ml) for 1 hour. C, untreated control. Data are mean values ± SD. The experiment was performed in triplicates and repeated twice. ***p<0.001 (two-way ANOVA).

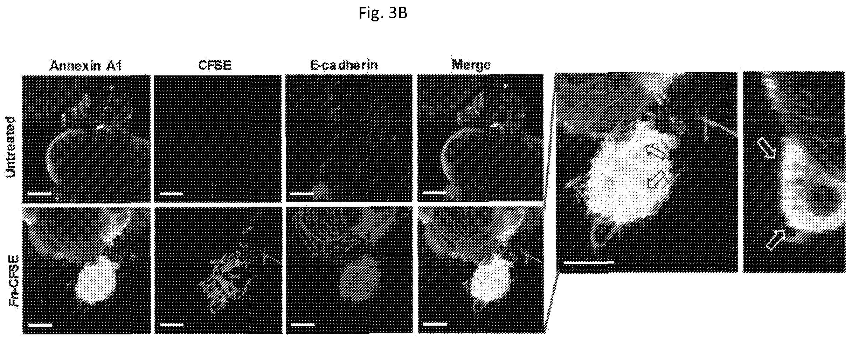

Fig. 3B are representative images of confocal microscopy analysis of 10C cells either untreated (top panel) or following incubation with CFSE-labeled *F*. *nucleatum* 12230 (red, bottom panel) and immuno-staining of Annexin A1 (green, appearing white on the image) and E-cadherin (blue, appearing gray in the image). Images were 1200x magnification. A side view of the enlarged image is shown on the far right. Note the enhanced expression of Annexin A1 in the *F. nucleatum-*bound cells and the co-localization of Annexin A1, E-cadherin and *F. nucleatum* on the cell membranes (arrows). The experiment was repeated more than three times. Scale bar, 500 nm.

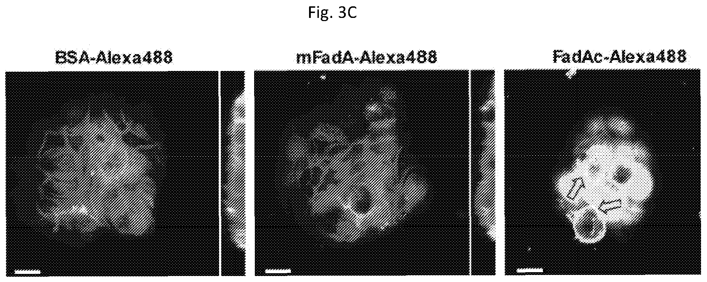

Fig. 3C are representative images of confocal microscopy analysis of 10C cells following incubation with Alexa Fluor^{™} 488-conjugated BSA, mFadA, or FadAc (300 µg/ml; red) and immuno-staining of Annexin A1 (green, appearing white on the image) and E-cadherin (blue, appearing gray in the image). Images were 1200x magnification. Note the enhanced expression of Annexin A1 and its co-localization with E-cadherin in response to FadAc (arrows), compared to BSA and mFadA. The experiment was repeated twice. The side views are shown to the right and bottom of each image. Scale bar, 500 nm.

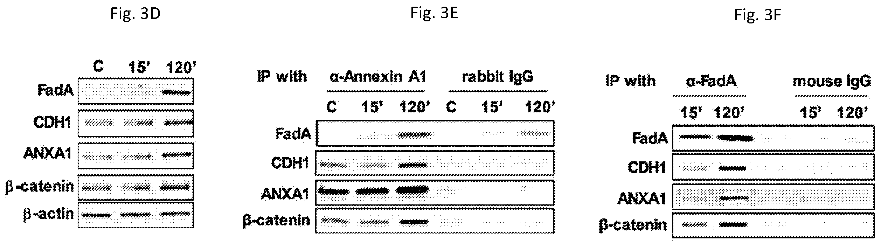

Fig. 3D shows Western blot analysis of FadA, E-cadherin (CDH1), Annexin A1 (ANXA1) and β-catenin in DLD1 cells following incubation with FadAc (1000 µg/ml) for 15 or 120 minutes. C, untreated cells. β-actin was included as internal control. The experiment was repeated three times.
Fig. 3E shows blots of co-immunoprecipitation with Annexin A1. DLD1 cell lysates were incubated with FadAc (1000 µg/ml) for 15 or 120 minutes and mixed with agarose beads conjugated with rabbit anti-Annexin A1 polyclonal antibody (α-Annexin A1) or control rabbit IgG. FadA, E-cadherin (CDH1), Annexin A1 and β-catenin in the eluates were detected by Western blot. C, untreated control. The experiment was repeated three times.
Fig. 3F shows blots of co-immunoprecipiation with FadA. DLD1 cell lysates were incubated with FadAc (1000 µg/ml) for 15 or 120 minutes and mixed with agarose beads conjugated with mouse anti-FadA monoclonal antibody (α-FadA) or control mouse IgG. FadA, E-cadherin (CDH1), Annexin A1 and β-catenin in the eluates were detected by Western blot. The experiment was repeated three times.

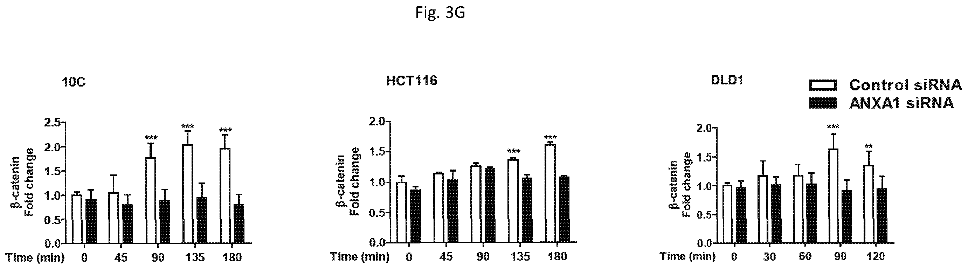

Fig. 3G are graphs of the results of flow cytometry analysis of β-catenin expression in 10C, HCT 116, and DLD1 cells following transfection with control siRNA (clear bars) or *ANXA1*-specific siRNA (solid bars) and subsequent incubation with *F*. *nucleatum* 12230 at MOI of approximately 20:1 for indicated time periods. The geometric means of cells treated with control siRNA at time 0 was designated as 1. Data are mean values ± SD. *** p<0.001. The experiment was performed in duplicates or triplicates and repeated 1-3 times. **p<0.01, ***p<0.001 (two-way ANOVA).

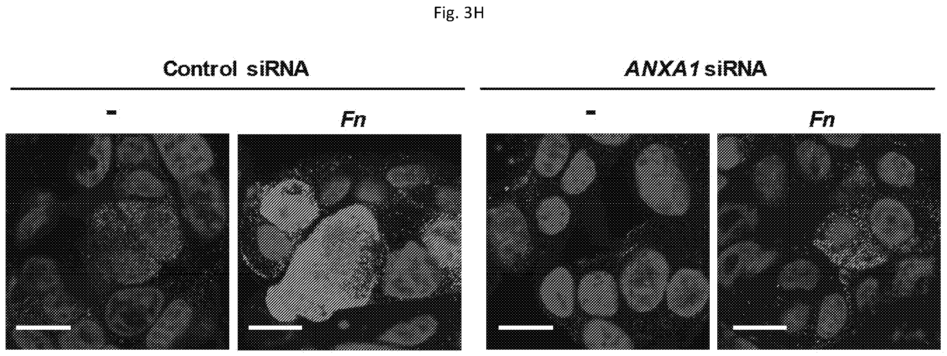

Fig. 3H shows representative images of immuno-staining of β-catenin in 10C cells following transfection with control siRNA or *ANXA1*-specific siRNA and subsequent incubation with *F. nucleatum* 12230 (Fn) at MOI of about 100:1 for 2 hours. β-catenin was stained with Alexa Fluor^{®}680 (red, showing as light gray in the image) and the nuclei with DAPI (blue, showing as dark gray in the image). The images were captured with confocal microscope at 800x magnification. -, no bacteria added. Note the increased expression of β-catenin and its nucleus translocation in response to *Fn* in control siRNA-treated cells, compared to *ANXA1* siRNA-treated cells.

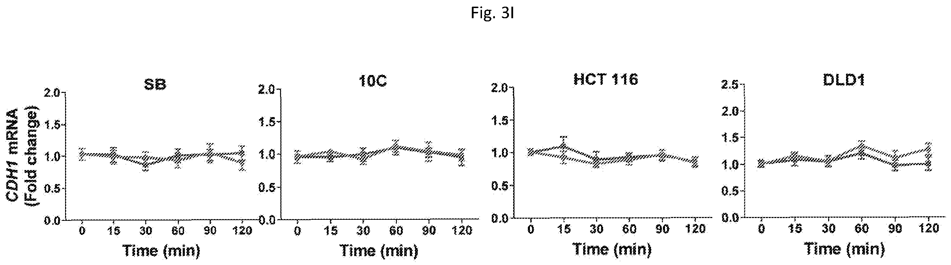

Fig. 3I shows the results of real time qPCR analysis of E-cadherin (CDH1) mRNA levels in SB, 10C, HCT116, and DLD1 cells following incubation with *F*. *nucleatum* 12230 (*Fn*) (dark lines with squares) or *fad*A-deletion mutant (US1) (light line with circles) for indicated time periods. All results were normalized to those of the untreated cells. The experiment was repeated twice. Scale bar, 200 nm.

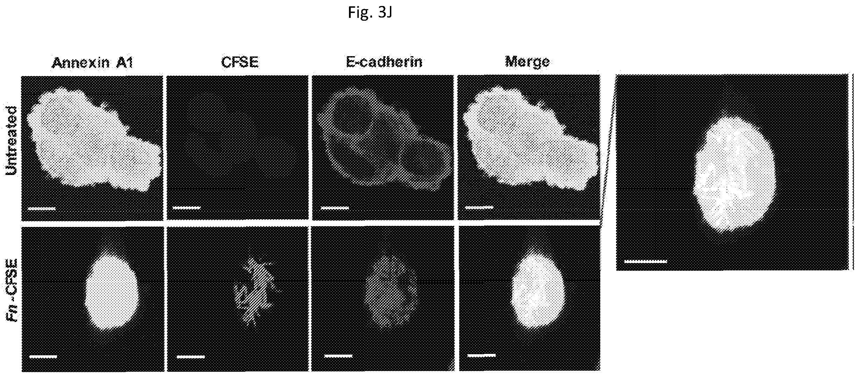

Fig. 3J are representative images of confocal microscopy analysis of DLD1 cells either untreated (top panel) or following incubation with CFSE-labeled *F*. *nucleatum* 12230 (red, bottom panel) and immuno-staining of Annexin A1 (green, appearing white on the image) and E-cadherin (blue, appearing gray in the image). Images were 1200x magnification. Arrows point to co-localization of Annexin A1, E-cadherin and Fn.
Fig. 3K shows the results of real-time qPCR analysis of cyclin D1 (*CCND1*) expression using mRNA extracted from the cancerous 10C, and human CRC cells HCT116 and RKO, each grown to 50% or 100% confluency. Data are mean values ± SEM. The experiment was performed in triplicates and repeated twice. *p<0.05, ***p<0.001 (student's t-test).
Fig. 3L shows Western-blot analysis of Cyclin D1, Annexin A1, and β-actin in RKO cells transfected with control vector or *ANXA1.* Induction of Cyclin D1 was observed in response to increased Annexin A1. The experiment was repeated twice.
**Figs. 4A****-4G** show FadA and Annexin A1 co-express in colorectal tumors in mice and human.
Fig. 4A is a plot and representative images of colorectal tumors generated in mice following treatment with PBS, *E. coli DH5α* (*E. coli*), *fadA*-deletion mutant US1 (US1) or *F*. *nucleatum* 12230 (*Fn*). Each symbol represents one mouse. Horizontal lines represent mean values. Representative tumors formed in the mouse colon are shown on the right, pointed by blue arrows on the right. *p<0.05, **p<0.01 (one-way ANOVA).
Fig. 4B is a plot of *ANXA1* mRNA levels in *APC*^{*min*/*+*} mouse colonic tumors (T) and normal colonic tissues (N) as measured by real-time qPCR. Each symbol represents one mouse. Horizontal lines represent mean values. *p<0.05, **p<0.01 (two-way ANOVA).
Fig. 4C is a plot showing the positive correlation between *fadA* gene copy numbers (x-axis) and *ANXA1* mRNA levels (y-axis) in *F*. *nucleatum-*induced *APC*^{*min*/*+*} mouse colonic tumors (n=34; Pearson's correlation). Each dot represents the average of qPCR results performed in duplicates.
Fig. 4D is a plot of the abundance of *fadA* in the paired normal and adenocarcinoma tissues from CRC patients (n=18) expressed as ratio of *fadA* over total 16S rRNA genes determined by qPCR. Each symbol represents one patient. Horizontal lines represent mean values. ** p<0.01 (paired t-test).
Fig. 4E is a plot of *ANXA1* mRNA levels in the paired normal and adenocarcinoma tissues from CRC patients measured by qPCR. Each symbol represents one patient. Horizontal lines represent mean values. ** p<0.01 (paired t-test).
Fig. 4F is a plot of the correlation between *fadA* gene copy numbers (x-axis) and *ANXA1* mRNA levels (y-axis) in human colorectal adenocarcinoma tissues (n=18; Pearson's correlation). Each dot represents the average of qPCR results performed in duplicates.

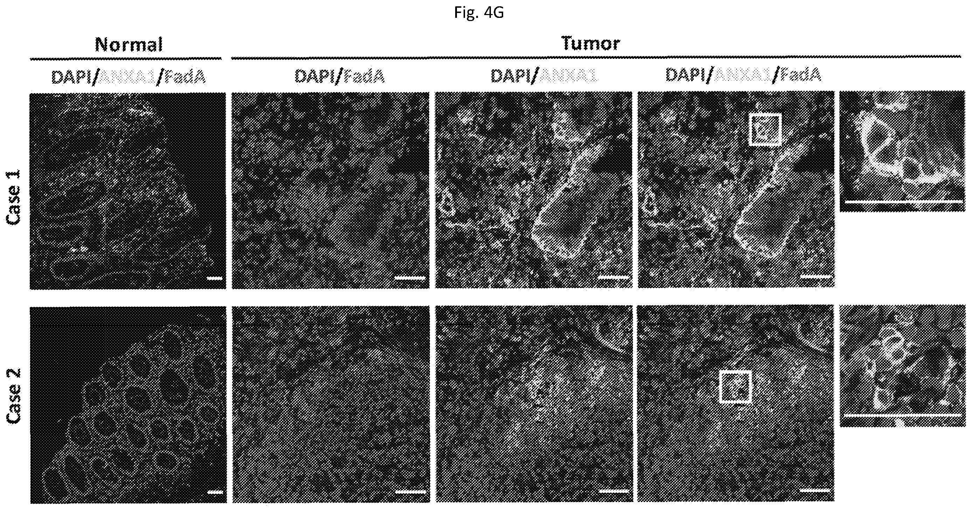

Fig 4G are representative images of confocal microscopy analysis of paired normal and carcinoma tissues from two colon cancer patients. The frozen sections were incubated with rabbit anti-AnnexinA1 polyclonal antibodies and 5G11 mouse anti-FadA monoclonal antibodies. The slides were then stained with Alexa Fluor^{®}680-conjugated donkey anti-rabbit and Alexa Fluor^{®}555-conjugated goat anti-mouse, washed, and covered in mounting medium containing DAPI. The scanning confocal microscopy mages were taken with a Nikon Ti Eclipse inverted microscope at 200x magnification for the normal tissues and 400x for the carcinomas. Scale bar, 50 µm. Colocalization of FadA (red) and Annexin A1 (green) was observed in carcinomas but not in the paired normal tissues.
**Figs. 5A****-5C** show that an anti-Annexin A1 antibody inhibits growth of human colorectal cancer cells.

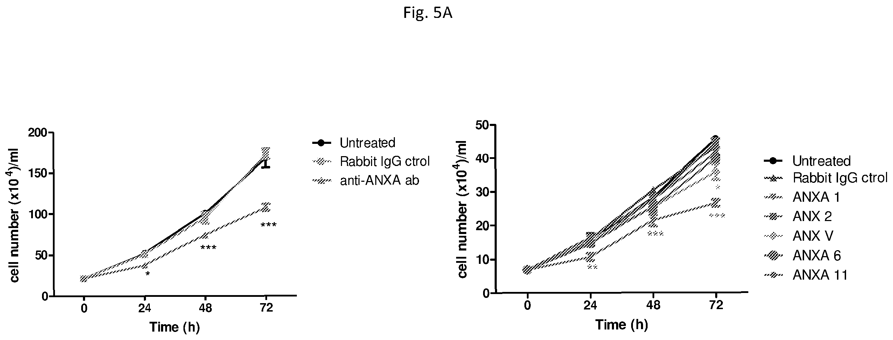

Fig. 5A shows graphs of the results of a cell proliferation assay of human CRC cell line HCT116 either untreated (black lines with circles) or following treatment with control rabbit IgG (gray lines with squares) or with various anti-Annexin antibodies including anti-Annexin A1 (red lines with triangles). Data are mean values ± SEM. The experiment was performed in triplicates and repeated three times. *p<0.05, ** p<0.01 and *** p<0.001, compared to the untreated cells (two-way ANOVA).

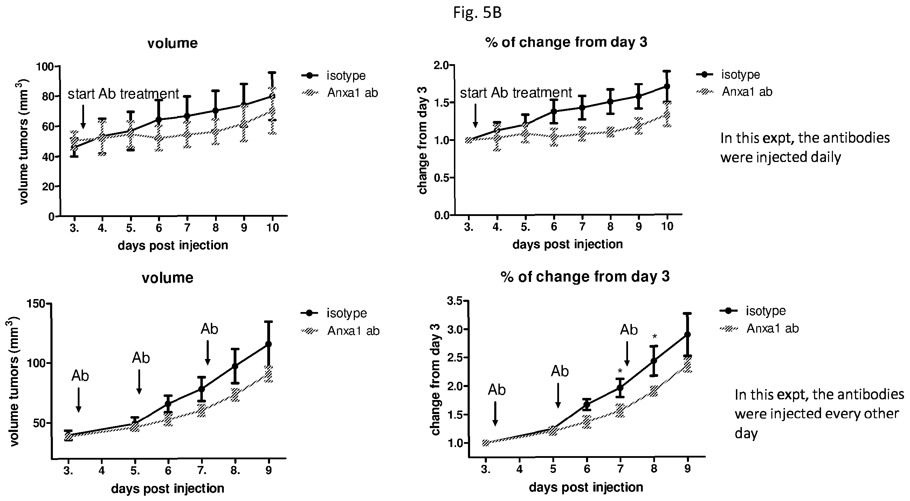

Fig. 5B are graphs of xenografted tumor growth in nude mice following subcutaneous and bilateral inoculation of HCT 116 cells and then treated every day (top graphs) or every other day other (bottom graphs) with an anti-Annexin A1 antibody or control. The tumor volumes were measured every day through day 10 and the percent change from day 3 to days 4-10 is also shown.

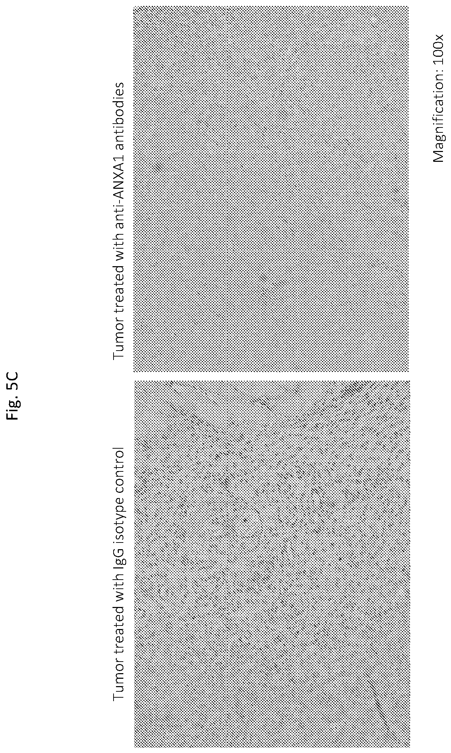

Fig. 5C are images of the cells immunostained for Annexin A1.

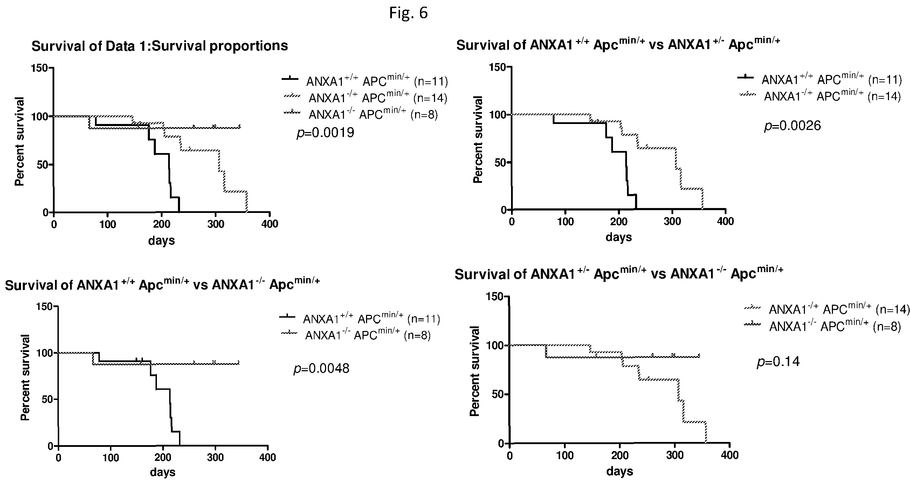

**Fig. 6** shows survival curves of various Annexin A1 knock-out mice crossed with APC mutant mice, including *ANXA1*^{*-*/}*⁻ APC*^{*min*/*+*}, *ANXA1*^{*+*/}*⁻ APC*^{*min*/}*⁺,* and *ANXA1*^{*+*/}*⁺ APC*^{*min*/}*⁺.* Survival curves are shown of all three mice; *ANXA1*^{*+*/}*⁺ APC*^{*min*/*+*} versus *ANXA1*^{*+*/}*⁻ APC*^{*min*/*+*}; *ANXA1*^{*+*/}*⁺ APC*^{*min*/*+*} versus *ANXA1*^{*-*/}*⁻ APC*^{*min*/*+*} ; and *ANXA1*^{*+*/}*⁻ APC*^{*min*/+}versus *ANXA1*^{*-*/}*⁻ APC*^{*min*/}*⁺.*

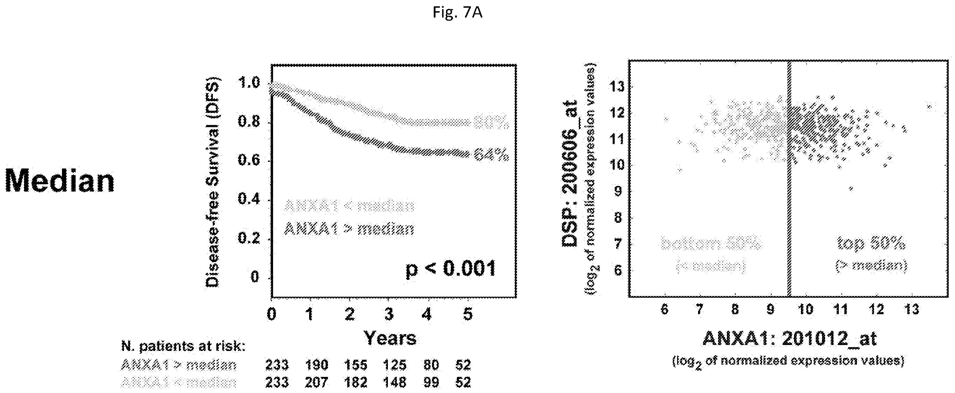

**Figs. 7A****-7C** show that Annexin A1 is a novel colon cancer prognosis marker.
   The relationship between *ANXA1* mRNA expression levels and DFS was investigated in a database of 466 primary colon carcinomas, assembled by pooling four independent gene-expression array datasets from the NCBI-GEO online repository (GSE14333, GSE17538, GSE31595, GSE37892), as previously described (Dalerba *et al.* 2016). The association between *ANXA1* expression levels and DFS was tested using Kaplan-Meier survival curves, after patient stratification in groups with high, medium and low ANXA1 expression, using three different methods.
Fig. 7A shows the results based on the median of *ANXA1* mRNA expression levels (high 50% vs. low 50%).

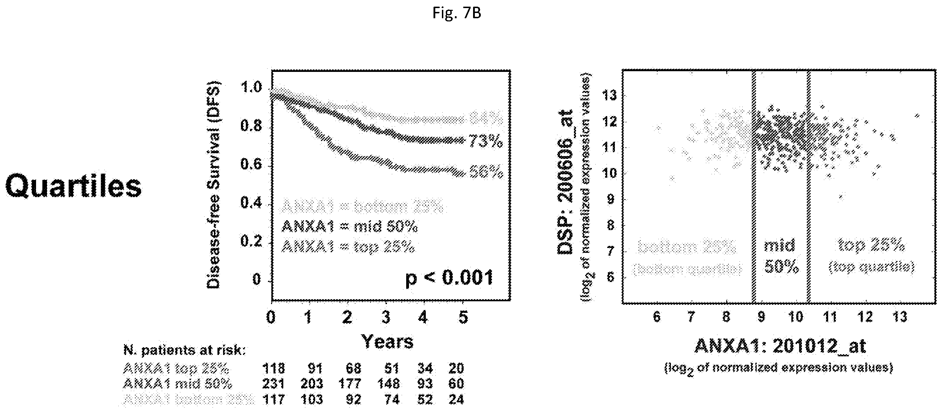

Fig. 7B shows the results based on the quartile distribution of *ANXA1* mRNA expression levels (high 25% vs. middle 50% vs. low 25%).

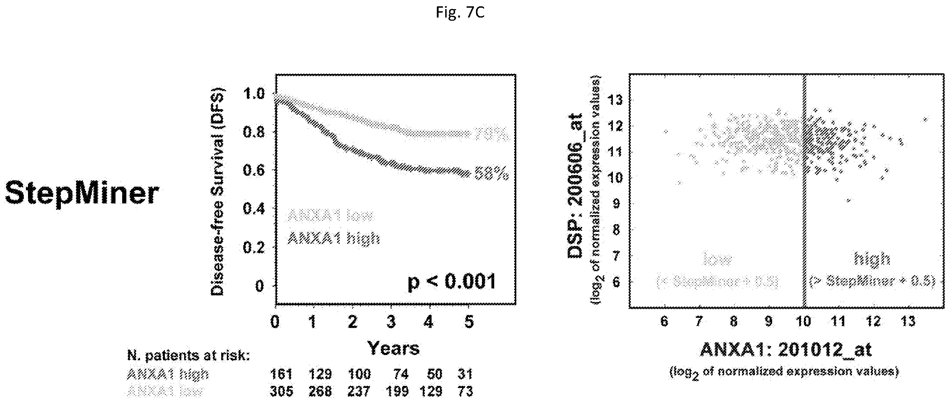

Fig. 7C shows the results based on *ANXA1* mRNA expression thresholds calculated using the StepMiner algorithm (low vs. high), as previously described (Dalerba *et al.* 2011; Sahoo *et al.* 2007). Overall, high ANXA1 mRNA expression levels were associated with a statistically significant reduction in DFS (p<0.001, log-rank test), irrespective of the method used for the stratification. Differences in *ANXA1* mRNA expression levels did not appear to correlate with differences in each tumor's relative content of epithelial cells in the analyzed biospecimens (*i.e.* tumor cell density) as revealed by the lack of visual correlations with the epithelial cell marker *Desmoplakin (DSP).*

### DETAILED DESCRIPTION OF THE INVENTION

### Definitions

The terms used in this specification generally have their ordinary meanings in the art, within the context of this invention and the specific context where each term is used. Certain terms are discussed below, or elsewhere in the specification, to provide additional guidance to the practitioner in describing the methods of the invention and how to use them. Moreover, it will be appreciated that the same thing can be said in more than one way. Consequently, alternative language and synonyms may be used for any one or more of the terms discussed herein, nor is any special significance to be placed upon whether or not a term is elaborated or discussed herein. Synonyms for certain terms are provided. A recital of one or more synonyms does not exclude the use of the other synonyms. The use of examples anywhere in the specification, including examples of any terms discussed herein, is illustrative only, and in no way limits the scope and meaning of the invention or any exemplified term. Likewise, the invention is not limited to its preferred embodiments.

The term "subject" as used in this application means an animal with an immune system such as avians and mammals. Mammals include canines, felines, rodents, bovine, equines, porcines, ovines, and primates. Avians include, but are not limited to, fowls, songbirds, and raptors. Thus, the invention can be used in veterinary medicine, *e.g*., to treat companion animals, farm animals, laboratory animals in zoological parks, and animals in the wild. The invention is particularly desirable for human medical applications.

The term "patient" as used in this application means a human subject. In some embodiments of the present invention, the "patient" is one suffering with cancer more specifically colorectal cancer, or FAP.

The terms "treat", "treatment", and the like refer to a means to slow down, relieve, ameliorate or alleviate at least one of the symptoms of the disease, or reverse the disease after its onset, preventing tumor growth, reducing tumor size, preventing or slowly the spread of metastasis, reversing (at least partially) chemo-resistance, and any other subjective or objective improvement in the patient related to the patient's cancer.

The terms "prevent", "prevention", and the like refer to acting prior to overt disease or disorder onset, to prevent the disease or disorder from developing or minimize the extent of the disease or disorder or slow its course of development.

The term "in need thereof" would be a subject known or suspected of having or being at risk of developing cancer, in particular, colorectal cancer.

A subject in need of treatment would be one that has already developed the disease or disorder. A subject in need of prevention would be one with risk factors of cancer, in particular colorectal cancer, including having FAP or a high level of Annexin A1 in the colorectal tissue indicating high risk of occurrence
The term "agent" as used herein means a substance that produces or is capable of producing an effect and would include, but is not limited to, chemicals, pharmaceuticals, biologics, small organic molecules, antibodies, nucleic acids, peptides, and proteins.

"Antibody," "fragment of an antibody," or "antibody fragment" are used interchangeably to mean one or more fragments or portions of an antibody that retain the ability to specifically bind to a specific antigen (Holliger et al., Nat. Biotech. (2005) 23(9): 1126). The present antibodies may be antibodies and/or fragments thereof. Antibody fragments include Fab, F(ab')2, scFv, disulfide linked Fv, Fc, or variants and/or mixtures. The antibodies may be chimeric, humanized, single chain, or bi-specific. All antibody isotypes are encompassed by the present disclosure, including, IgA, IgD, IgE, IgG, and IgM. Suitable IgG subtypes include IgG1, IgG2, IgG3 and IgG4. An antibody light or heavy chain variable region consists of a framework region interrupted by three hypervariable regions, referred to as complementarity determining regions (CDRs). The CDRs of the present antibodies or antigen-binding portions can be from a non-human or a human source. The framework of the present antibodies or antigen-binding portions can be human, humanized, non-human (*e.g*., a murine framework modified to decrease antigenicity in humans), or a synthetic framework (*e.g*., a consensus sequence).

The terms "therapeutically effective amount" or "effective amount" encompasses an amount sufficient to ameliorate or prevent a symptom or sign of the medical condition. Effective amount also means an amount sufficient to allow or facilitate diagnosis. An effective amount for a particular subject may vary depending on factors such as the condition being treated, the overall health of the patient, the method route and dose of administration and the severity of side effects. An effective amount can be the maximal dose or dosing protocol that avoids significant side effects or toxic effects.

The terms "cancer", "tumor", "cancerous", and "malignant" refer to or describe the physiological condition in mammals that is typically characterized by unregulated cell growth. Examples of cancer include but are not limited to, carcinoma including adenocarcinoma, lymphoma, blastoma, melanoma, sarcoma, and leukemia. More particular examples of such cancers include melanoma, lung cancer, head and neck cancer, renal cell cancer, colon cancer, colorectal cancer, squamous cell cancer, small-cell lung cancer, non-small cell lung cancer, gastrointestinal cancer, Hodgkin's and non-Hodgkin's lymphoma, pancreatic cancer, glioblastoma, glioma, cervical cancer, ovarian cancer, liver cancer such as hepatic carcinoma and hepatoma, bladder cancer, breast cancer, endometrial carcinoma, myeloma (such as multiple myeloma), salivary gland carcinoma, kidney cancer such as renal cell carcinoma and Wilms' tumors, basal cell carcinoma, prostate cancer, vulval cancer, thyroid cancer, testicular cancer, and esophageal cancer.

A "tumor" refers to the mass of tissue formed as cancerous cells grow and multiply, which can invade and destroy normal adjacent tissues. Cancer cells can break away from a malignant tumor and enter the bloodstream or lymphatic system, such that cancer cells spread from the primary tumor to form new tumors in other organs.

The term "about" or "approximately" means within an acceptable error range for the particular value as determined by one of ordinary skill in the art, which will depend in part on how the value is measured or determined, *i.e.,* the limitations of the measurement system, *i.e.,* the degree of precision required for a particular purpose, such as a pharmaceutical formulation. For example, "about" can mean within 1 or more than 1 standard deviations, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated, the term "about" meaning within an acceptable error range for the particular value should be assumed.

### Abbreviations

- MOI:: multiplicity of infection
- CRC:: colon or colorectal cancer
- FAP:: familial adenomatous polyposis
- *Fn:*: *Fusobacterium nucleatum* or *F*. *nucleatum*
- *ANXA1:*: Annexin A1
- *CDH1:*: E-cadherin

### Annexin A1 as a Target for Treatment and Prevention of Cancer as well as a Biomarker

The instant invention is based, in part, on the discovery that *Fn* selectively binds to proliferating CRC cells that express Annexin A1, and *Fn* binding in turn, induces further Annexin A1 expression in an E-cadherin dependent manner. Induced Annexin A1 forms a complex with FadA, E-cadherin and β-catenin, which is required for activating β-catenin and stimulating CRC growth.

A positive correlation between FadA and Annexin A1 expression was observed in colorectal tumors in mice and humans. Thus, according to the present invention, Annexin A1 has been identified as a critical, yet previously unrecognized, component in *Fn*-induced Wnt/β-catenin signaling. Approximately 75% of CRC are caused by mutations in the Wnt/β-catenin pathway. However, Wnt/β-catenin signaling is involved in a broad spectrum of cellular functions. Due to such complexity, so far no Wnt inhibitors have received FDA approval for cancer treatment. Similarly, E-cadherin, through which *F. nucleatum* stimulates CRC, is also ubiquitous, rendering it an unsuitable therapeutic target, either. In contrast, Annexin A1, due to its selective expression in proliferating cancerous cells, is a promising therapeutic target. Inhibition of Annexin A1 suppresses β-catenin signaling in cancerous cells without affecting the noncancerous cells, thus may have less adverse "off-target" effects.

*Fn* causes chemo-resistance in CRC and promotes metastasis (Bullman *et al.* 2017; Yu *et al.* 2017), imposing a significant challenge to treatment. Antibiotic treatment is not desirable due to disturbance of the normal intestinal flora. Thus, there exists a need to prevent and/or treat *Fn* and *Fn*-related adverse effects in patients with CRC. This instant invention addresses these needs.

According to the present invention, Annexin A1 is a novel therapeutic target for cancer treatment.

Annexin A1 belongs to the Annexin family of Ca2+-dependent phospholipid-binding proteins, with a molecular weight of 35-40 KD, and is present in both cytoplasma and membrane. Annexin A1 has been suggested to play a role in resolution of inflammation (Peretti *et al.* 2009). Annexin A1 has been postulated to be either a tumor suppressor or promoter depending on tumor type (Guo *et al.* 2013; Boudhraa *et al.* 2016). Although Annexin A1 has been associated with CRC (Onozawa *et al.* 2017; Su *et al.* 2010), its role in CRC was unclear.

Shown herein, is the fundamental mechanistic difference between *F. nucleatum* interaction with the cancerous and non-cancerous cells. *F*. *nucleatum* preferentially binds to the cancerous cells, aided by Annexin A1, which is specifically expressed in proliferating CRC cells.

This is consistent with the inventors' previous report that, although *F. nucleatum* can be detected in both colorectal adenoma and adenocarcinoma tissues, the *fad*A gene levels are significantly higher in the latter than the former (Rubinstein *et al.* 2013). Whereas *F*. *nucleatum* may not alter pre-cancerous cells to cancer, once the benign cells become cancerous, they express elevated levels of Annexin A1, through which *F*. *nucleatum* activates the Wnt/β-catenin signaling and stimulates growth.

Based on these results, a "two-hit" model in colorectal cancer is hypothesized, in which the accumulation of driver somatic mutation(s), *e.g*, increased expression of Annexin A1, serving as the first "hit", and microbes, *e.g. F. nucleatum,* as the second "hit", exacerbating cancer progression. This model extends from the well-accepted "adenoma-carcinoma" model (Fearon and Vogelstein 1990) and identifies microbes as facilitators for colorectal carcinogenesis. In support of the "two-hit" model, studies have consistently found that microbes promote cancer in predisposed hosts, *i.e.* after "first hit" occurs.

In the case of *F*. *nucleatum,* stimulation of CRC is through E-cadherin-mediated, positive feedback loop of FadA and Annexin A1 identified in the cancerous cells, which is absent in the non-cancerous cells. Increased expression of Annexin A1 in proliferating cancer cells enhances *F. nucleatum* binding, which in turn stimulates Annexin A1 expression and further enhances *F. nucleatum* binding activating β-catenin signaling.

Also shown herein is that Annexin A1 is a novel biomarker for colon cancer recurrence, independent of cancer stage, grade, age and sex. Annexin A1 may be used in combination with cancer stage to improve the prognostic stratification of colon cancer patients.

Additionally shown herein is that inhibiting Annexin A1 in mice with the same mutation as patients with FAP (the APC gene) prolonged the life of these mice.

In short, this is the first study detailing the molecular mechanism of Annexin A1 in cancer and the first elucidation of its interaction with cancer stimulating microorganism. Given the broad implication of Wnt/β-catenin in cancer and increasing numbers of reports of *F. nucleatum* in different types of cancer, Annexin A1 is a novel therapeutic target for different types of cancers implicated with *F*. *nucleatum.* In addition, although Annexin A1 was identified through its interaction with *F*. *nucleatum,* it has been demonstrated herein that it is a CRC growth factor independent of the microorganism. Its expression in other cancer types has also been detected (Fig. 1K).

### Methods and Compositions for Treating and Preventing Cancer

As shown herein, Annexin A1 is a viable target for the treatment of cancer. Also shown herein the use of Annexin A1 specific short interfering RNA or siRNA inhibited the growth of cancerous cells including colorectal cancer cells.

SiRNA is a double-stranded RNA molecule, about 20-25 base pairs in length that can interfere with expression with complementary nucleotide sequences by degrading mRNA after transcription.

Additionally, microRNAs or miRNAs small non-coding RNAs averaging 22 nucleotides that regulate the expression of their target mRNA transcripts can also be used. MiRNA works by binding to the 3'UTR of a gene, in this case, *ANXA1.*

Thus, disclosed herein is a method of treating and/or preventing cancer, especially colorectal cancer, in a subject in need thereof by administering an Annexin A1 specific short interfering RNA.

Disclosed herein is a method of treating and/or preventing cancer, especially colorectal cancer, in a subject in need thereof by administering an Annexin A1 specific microRNA.

Disclosed herein is a method of treating and/or preventing cancer, especially colorectal cancer, in a subject in need thereof by administering DNA which encodes an Annexin A1 specific short interfering RNA.

Disclosed herein is a method of treating and/or preventing cancer, especially colorectal cancer, in a subject in need thereof by administering DNA which encodes an Annexin A1 specific microRNA.

Disclosed herein is a method of preventing colorectal cancer in a subject with familial adenomatous polyposis (FAP) by administering an Annexin A1 specific short interfering RNA.

Disclosed herein is a method of preventing colorectal cancer in a subject with familial adenomatous polyposis (FAP) by administering an Annexin A1 specific microRNA.

Disclosed herein is a method of preventing colorectal cancer in a subject with familial adenomatous polyposis (FAP) by administering DNA which encodes an Annexin A1 specific short interfering RNA.

Disclosed herein is a method of preventing colorectal cancer in a subject with familial adenomatous polyposis (FAP) by administering DNA which encodes an Annexin A1 microRNA.

Disclosed herein is a method of reducing chemo-resistance of cancer in a subject in need thereof comprising administering an Annexin A1 specific short interfering RNA.

Disclosed herein is a method of reducing chemo-resistance of cancer in a subject in need thereof comprising administering an Annexin A1 microRNA.

Disclosed herein is a method of reducing chemo-resistance of cancer in a subject in need thereof comprising administering DNA which encodes an Annexin A1 specific short interfering RNA.

Disclosed herein is a method of reducing chemo-resistance of cancer in a subject in need thereof comprising administering DNA which encodes an Annexin A1 specific short interfering RNA.

Disclosed herein is a method for inhibiting cancer cell proliferation, which comprises reducing Annexin A1 expression on the cancer cells by contacting the cells with an Annexin A1 specific short interfering RNA.

Disclosed herein is a method for inhibiting cancer cell proliferation, which comprises reducing Annexin A1 expression on the cancer cells by contacting the cells with an Annexin A1 microRNA.

Disclosed herein is a method for inhibiting cancer cell proliferation, which comprises reducing Annexin A1 expression on the cancer cells by contacting the cells with DNA which encodes an Annexin A1 specific short interfering RNA.

Disclosed herein is a method for inhibiting cancer cell proliferation, which comprises reducing Annexin A1 expression on the cancer cells by contacting the cells with DNA which encodes an Annexin A1 specific short interfering RNA.

An Annexin A1 specific siRNA or miRNA that binds to the Annexin A1 mRNA or mRNA 3'UTR or the DNA that encodes such RNA can be designed by using the sequence information.

The sequence for the *ANXA1* gene can be found on the National Center for Biotechnology Database and can be used to manufacture the interfering RNA molecules by methods known in the art. Annexin A1 is encoded by the *ANXA1* gene on chromosome 9 (Gene ID: 301).

The siRNA or microRNAor DNA can be made by recombinant methods known in the art. The siRNA or microRNA or DNA can also be modified for increasing other desirable properties, such as increased stability, decreased degradation in the body, and increased cellular uptake. Additionally, mi196a has been shown to downregulate Annexin A1 (see Luthra *et al.* 2008).

It may be that the RNA or DNA would be targeted to particular tissues or cells. Optionally, the tissue is cancer and the cells are cancer cells.

One such method for delivering the nucleic acids is receptor mediated endocytosis where the nucleic acid is coupled to a targeting molecule that can bind to a specific cell surface receptor, inducing endocytosis and transfer of the nucleic acid into cells. Coupling is normally achieved by covalently linking poly-lysine to the receptor molecule and then arranging for (reversible) binding of the negatively charged nucleic acid to the positively charged poly-lysine component.

Another approach utilizes the transferrin receptor or folate receptor which is expressed in many cell types. For example, when producing the microRNA for this method of administration, the microRNA could be manufactured to have a guide strand which is identical to the microRNA of interest and a passenger strand that is modified and linked to a molecule for increasing cellular uptake.

Another method to administer the nucleic acids to the proper tissue is direct injection/particle bombardment, where the nucleic acid is to be injected directly with a syringe and needle into a specific tissue, such as cancer tissue.

An alternative direct injection approach uses particle bombardment ('gene gun') techniques: nucleic acid is coated on to metal pellets and fired from a special gun into cells. Successful gene transfer into a number of different tissues has been obtained using this approach. Such direct injection techniques are simple and comparatively safe.

Another method for delivery of microRNA and siRNA and DNA to the proper tissue or cell is by using adeno-associated viruses (AAV). Nucleic acid delivered in these viral vectors is continually expressed, replacing the expression of the microRNA or siRNA or DNA that is not expressed in the subject. Also, AAV have different serotypes allowing for tissue-specific delivery due to the natural tropism toward different organs of each individual AAV serotype as well as the different cellular receptors with which each AAV serotype interacts. The use of tissue-specific promoters for expression allows for further specificity in addition to the AAV serotype.

Other mammalian virus vectors that can be used to deliver the RNA or DNA include oncoretroviral vectors, adenovirus vectors, Herpes simplex virus vectors, and lentiviruses.

Delivery vehicles such as liposomes, nanocapsules, nanoparticles, microparticles, microspheres, lipid particles, vesicles, and the like, may be used for the introduction of the compositions into suitable host cells. In particular, vector delivered transgenes or proteins may be formulated for delivery either encapsulated in a lipid particle, a liposome, a vesicle, a nanosphere, or a nanoparticle or the like.

The formation and use of liposomes is generally known to those of skill in the art. Recently, liposomes were developed with improved serum stability and circulation half-times (U.S. Patent No. 5,741,516). Further, various methods of liposome and liposome like preparations as potential drug carriers have been described (U.S. Patent Nos. 5,567,434; 5,552,157; 5,565,213; 5,738,868; and 5,795,587).

Liposomes have been used successfully with a number of cell types that are normally resistant to transfection by other procedures. In addition, liposomes are free of the DNA length constraints that are typical of viral-based delivery systems. Liposomes have been used effectively to introduce genes, drugs, radiotherapeutic agents, viruses, transcription factors and allosteric effectors into a variety of cultured cell lines and animals. In addition, several successful clinical trials examining the effectiveness of liposome-mediated drug delivery have been completed.

Liposomes are formed from phospholipids that are dispersed in an aqueous medium and spontaneously form multilamellar concentric bilayer vesicles (also termed multilamellar vesicles (MLVs). MLVs generally have diameters of from 25 nm to 4 µm. Sonication of MLVs results in the formation of small unilamellar vesicles (SUVs) with diameters in the range of 200 to 500Å, containing an aqueous solution in the core.

Alternatively, nanocapsule formulations may be used. Nanocapsules can generally entrap substances in a stable and reproducible way.

Nanoparticles are a colloidal carrier system that has been shown to improve the efficacy of an encapsulated drug by prolonging the serum half-life. Polyalkylcyanoacrylates (PACAs) nanoparticles are a polymer colloidal drug delivery system that is in clinical development (described, for example, by Stella et al. (2000) J. Pharm. Sci., 89: 1452-1464; Brigger et al. (2001) Int. J. Pharm 214: 37-42; Calvo et al. (2001) Pharm. Res. 18: 1157-1166; and Li et al. (2001) Biol. Pharm. Bull. 24: 662-665). Biodegradable poly(hydroxyl acids), such as the copolymers of poly(lactic acid) (PLA) and poly(lactic-co-glycolide) (PLGA) are being extensively used in biomedical applications and have received FDA approval for certain clinical applications. In addition, nanoparticles have many desirable carrier features including (i) that the agent to be encapsulated comprises a reasonably high weight fraction (loading) of the total carrier system; (ii) that the amount of agent used in the first step of the encapsulation process is incorporated into the final carrier (entrapment efficiency) at a reasonably high level; (iii) that the carrier has the ability to be freeze-dried and reconstituted in solution without aggregation; (iv) that the carrier be biodegradable; (v) that the carrier system be of small size; and (vi) that the carrier enhances the particles persistence. Nanoparticles may be synthesized using virtually any biodegradable shell known in the art. Such polymers are biocompatible and biodegradable and are subject to modifications that desirably increase the photochemical efficacy and circulation lifetime of the nanoparticle. The polymer may be modified with a terminal carboxylic acid group (COOH) that increases the negative charge of the particle and thus limits the interaction with negatively charged nucleic acids. Nanoparticles may also be modified with polyethylene glycol (PEG), which also increases the half-life and stability of the particles in circulation. Alternatively, the COOH group may be converted to an N-hydroxysuccinimide (NHS) ester for covalent conjugation to amine-modified compounds.

Additionally shown herein anti-Annexin A1 antibodies inhibited the growth of colorectal cancer cells.

Thus, disclosed herein is a method of treating and/or preventing cancer, especially colorectal cancer, in a subject in need thereof by administering an anti-Annexin A1 antibody, minibody, Fab or fragment, camelid, or nanobody.

Disclosed herein is a method of preventing colorectal cancer in a subject with familial adenomatous polyposis (FAP) by administering an anti-Annexin A1 antibody minibody, Fab or fragment, camelid, or nanobody.

Disclosed herein is a method of reducing chemo-resistance of cancer in a subject in need thereof comprising administering an anti-Annexin A1 antibody minibody, Fab or fragment, camelid, or nanobody.

Disclosed herein is a method for inhibiting cancer cell proliferation, which comprises reducing Annexin A1 expression on the cancer cells by contacting the cells with an anti-Annexin A1 antibody minibody, Fab or fragment, camelid, or nanobody.

An anti-Annexin A1 antibody that binds to the Annexin A1 can be designed by using the sequence information and a conventional method, for example, the hybridoma technology or recombinant technology. Antigen-binding fragments of an intact antibody (full-length antibody) can be prepared via routine methods. For example, F(ab')2 fragments can be produced by pepsin digestion of an antibody molecule, and Fab fragments that can be generated by reducing the disulfide bridges of F(ab')2 fragments.

Genetically engineered antibodies, such as humanized antibodies, chimeric antibodies, single-chain antibodies, and bi-specific antibodies, can be produced via, *e.g.,* conventional recombinant technology.

The sequence for the *ANXA1* gene and protein can be found on the National Center for Biotechnology Database and can be used to manufacture the interfering RNA molecules and antibodies by methods known in the art. Annexin A1 is encoded by the *ANXA1* gene on chromosome 9 (Gene ID: 301).

Other agents can be used to block or inhibit Annexin A1 including small molecules.

Thus, disclosed herein is a method of treating and/or preventing cancer, especially colorectal cancer, in a subject in need thereof by administering a small molecule that blocks or inhibits Annexin A1.

Disclosed herein is a method of preventing colorectal cancer in a subject with familial adenomatous polyposis (FAP) by administering a small molecule that blocks or inhibits Annexin A1.

Disclosed herein is a method of reducing chemo-resistance of cancer in a subject in need thereof comprising administering a small molecule that blocks or inhibits Annexin A1.

Disclosed herein is a method for inhibiting cancer cell proliferation, which comprises reducing Annexin A1 expression on the cancer cells by contacting the cells with a small molecule that blocks or inhibits Annexin A1.

Optionally, the small molecule includes but is not limited to indomethacin, eurycomanone, and sorafenib and associated structural analogs and derivatives.

All of these agents can be used alone, in combination with each other and/or in combination with other therapeutic agents.

All of the agents discussed herein can be in the form of pharmaceutical compositions.

Disclosed herein is a pharmaceutical composition comprising an Annexin A1 specific siRNA and a pharmaceutically acceptable, diluent, carrier or adjuvant. Disclosed herein is a pharmaceutical composition comprising: an Annexin A1 specific siRNA; a vector, liposome, nanocapsule, nanoparticle, microparticle, microsphere, lipid particle, or vesicle; and a pharmaceutically acceptable, diluent, carrier or adjuvant.

Disclosed herein is a pharmaceutical composition comprising an Annexin A1 specific miRNA and a pharmaceutically acceptable, diluent, carrier or adjuvant. Disclosed herein is a pharmaceutical composition comprising an Annexin A1 specific miRNA; a vector, liposome, nanocapsule, nanoparticle, microparticle, microsphere, lipid particle, or vesicle; and a pharmaceutically acceptable, diluent, carrier or adjuvant.

Disclosed herein is a pharmaceutical composition comprising DNA which encodes an Annexin A1 specific siRNA and a pharmaceutically acceptable, diluent, carrier or adjuvant. Disclosed herein is a pharmaceutical composition comprising: DNA which encodes an Annexin A1 specific siRNA; a vector, liposome, nanocapsule, nanoparticle, microparticle, microsphere, lipid particle, or vesicle; and a pharmaceutically acceptable, diluent, carrier or adjuvant.

Disclosed herein is a pharmaceutical composition comprising DNA which encodes of an Annexin A1 specific miRNA and a pharmaceutically acceptable, diluent, carrier or adjuvant. Disclosed herein is a pharmaceutical composition comprising an Annexin A1 specific miRNA; a vector, liposome, nanocapsule, nanoparticle, microparticle, microsphere, lipid particle, or vesicle; and a pharmaceutically acceptable, diluent, carrier or adjuvant.

Disclosed herein is a pharmaceutical composition comprising an anti-Annexin A1 antibody, minibody, Fab or fragment, camelid, or nanobody and a pharmaceutically acceptable, diluent, carrier or adjuvant.

Disclosed herein is a pharmaceutical composition comprising a small molecule that blocks or inhibits Annexin A1 chosen from the group consisting of indomethacin, eurycomanone, and sorafenib and associated structural analogs and derivatives and a pharmaceutically acceptable, diluent, carrier or adjuvant.

Most preferred methods of administration of the agents and compositions for use in the methods disclosed are oral, intrathecal, nasal, and parental including intravenous. The pharmacological agent must be in the appropriate form for administration of choice.

Such pharmaceutical compositions comprising one or more pharmacological agents for administration may comprise a therapeutically effective amount of the pharmacological agent and a pharmaceutically acceptable carrier.

The phrase "pharmaceutically acceptable" as used herein refers to molecular entities and compositions that are physiologically tolerable and do not typically produce an allergic or similar untoward reaction, such as gastric upset, dizziness and the like, when administered to a human, and approved by a regulatory agency of the Federal or a state government or listed in the U.S. Pharmacopeia or other generally recognized pharmacopeia for use in animals, and more particularly in humans.

The term "carrier" refers to a diluent, adjuvant, excipient, or vehicle with which the therapeutic is administered. Such pharmaceutical carriers can be sterile liquids, such as saline solutions in water and oils, including those of petroleum, animal, vegetable, or synthetic origin, such as peanut oil, soybean oil, mineral oil, sesame oil, and the like. A saline solution is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. The composition, if desired, can also contain minor amounts of wetting or emulsifying agents, or pH buffering agents. Adjuvants can also be added to the RNA to protect it from degradation.

Pharmaceutical compositions adapted for oral administration may be capsules, tablets, powders, granules, solutions, syrups, suspensions (in non-aqueous or aqueous liquids), or emulsions. Tablets or hard gelatin capsules may comprise lactose, starch or derivatives thereof, magnesium stearate, sodium saccharine, cellulose, magnesium carbonate, stearic acid or salts thereof. Soft gelatin capsules may comprise vegetable oils, waxes, fats, semi-solid, or liquid polyols. Solutions and syrups may comprise water, polyols, and sugars. An active agent intended for oral administration may be coated with or admixed with a material that delays disintegration and/or absorption of the active agent in the gastrointestinal tract. Thus, the sustained release may be achieved over many hours and if necessary, the active agent can be protected from degradation within the stomach. Pharmaceutical compositions for oral administration may be formulated to facilitate release of an active agent at a particular gastrointestinal location due to specific pH or enzymatic conditions.

Pharmaceutical compositions adapted for nasal and pulmonary administration may comprise solid carriers such as powders, which can be administered by rapid inhalation through the nose. Compositions for nasal administration may comprise liquid carriers, such as sprays or drops. Alternatively, inhalation directly through into the lungs may be accomplished by inhalation deeply or installation through a mouthpiece. These compositions may comprise aqueous or oil solutions of the active ingredient. Compositions for inhalation may be supplied in specially adapted devices including, but not limited to, pressurized aerosols, nebulizers or insufflators, which can be constructed so as to provide predetermined dosages of the active ingredient.

A further preferred form of administration is parenteral including intravenous administration. Pharmaceutical compositions adapted for parenteral administration, including intravenous administration, include aqueous and non-aqueous sterile injectable solutions or suspensions, which may contain anti-oxidants, buffers, bacteriostats, and solutes that render the compositions substantially isotonic with the blood of the subject. Other components which may be present in such compositions include water, alcohols, polyols, glycerine, and vegetable oils. Compositions adapted for parental administration may be presented in unit-dose or multi-dose containers, such as sealed ampules and vials, and may be stored in a freeze-dried (lyophilized) condition requiring only the addition of a sterile carrier, immediately prior to use. Extemporaneous injection solutions and suspensions may be prepared from sterile powders, granules, and tablets. Suitable vehicles that can be used to provide parenteral dosage forms of the invention are well known to those skilled in the art. Examples include: Water for Injection USP; aqueous vehicles such as Sodium Chloride Injection, Ringer's Injection, Dextrose Injection, Dextrose and Sodium Chloride Injection, and Lactated Ringer's Injection; water-miscible vehicles such as ethyl alcohol, polyethylene glycol, and polypropylene glycol; and non-aqueous vehicles such as corn oil, cottonseed oil, peanut oil, sesame oil, ethyl oleate, isopropyl myristate, and benzyl benzoate.

Further methods of administration include sublingual, vaginal, buccal, or rectal; or transdermal administration to a subject.

Selection of a therapeutically effective dose will be determined by the skilled artisan considering several factors, which will be known to one of ordinary skill in the art. Such factors include the particular form of the pharmacological agent, and its pharmacokinetic parameters such as bioavailability, metabolism, and half-life, which will have been established during the usual development procedures typically employed in obtaining regulatory approval for a pharmaceutical compound. Further factors in considering the dose include the condition or disease to be treated or the benefit to be achieved in a normal individual, the body mass of the patient, the route of administration, whether the administration is acute or chronic, concomitant medications, and other factors well known to affect the efficacy of administered pharmaceutical agents. Thus, the precise dose should be decided according to the judgment of the person of skill in the art, and each patient's circumstances, and according to standard clinical techniques.

As used herein, the terms "therapeutically effective amount", "therapeutically effective dose" and "effective amount" refer to an amount of RNA, antibody or small molecule, or other agents and compositions which inhibit *ANXA1* that, when administered alone or in combination with an additional therapeutic agent to a cell, tissue, or subject, is effective to cause a measurable improvement in one or more symptoms of a disease or condition or the progression of such disease or condition, e.g. solid tumor cancers. A therapeutically effective dose further refers to that amount of the compound sufficient to result in at least partial amelioration of symptoms, e.g., treatment, healing, prevention or amelioration of the relevant medical condition, or an increase in rate of treatment, healing, prevention or amelioration of such conditions. When applied to an individual active ingredient administered alone, a therapeutically effective dose refers to that ingredient alone. When applied to a combination, a therapeutically effective dose refers to combined amounts of the active ingredients that result in the therapeutic effect, whether administered in combination, serially or simultaneously. An effective amount can also result in an improvement in a subjective measure in cases where subjective measures are used to assess disease severity.

### Combination Methods and Compositions for Treating and Preventing Cancer

Also disclosed herein are methods and compositions for the treatment and prevention of cancer wherein an agent which inhibits or blocks Annexin A1 is used in combination with a therapeutically effective amount of other therapeutic agents including but not limited to chemotherapeutic agents, targeted chemotherapeutic agents and immunotherapy.

Disclosed herein are methods and compositions to reduce chemo-resistance of cancer wherein an agent which inhibits or blocks Annexin A1 is used in combination with a therapeutically effective amount of other therapeutic agents including but not limited to chemotherapeutic agents.

The agent which inhibits or blocks Annexin A1 may improve the efficacy and cause the cancer to become more susceptible to other treatments, including but not limited to chemotherapeutic agents, targeted therapeutic agents and immunotherapy.

As discussed herein, *Fn* causes chemo-resistance in CRC and promotes metastasis (Bullman *et al.* 2017; Yu *et al.* 2017), imposing a significant challenge to treatment. Antibiotic treatment is not desirable due to disturbance of the normal intestinal flora. Also as shown herein Annexin A1 and *Fn* work together in the pathogenesis of cancer. Thus, targeting Annexin A1 would increase the effectiveness of other cancer therapeutics.

Thus, also disclosed is a method of treating cancer in a subject in need thereof comprising administering a therapeutically effective amount of an agent which inhibits or blocks Annexin A1 and a therapeutically effective amount of an additional therapeutic agent, including but not limited to a chemotherapeutic agent, a targeted therapeutic agent and immunotherapy and combinations thereof.

A "chemotherapeutic agent" or "chemotherapeutic drug" is a chemical compound useful in the treatment of cancer, regardless of mechanism of action. Classes of chemotherapeutic agents include, but are not limited to, microtubule-targeting moietys (MTAs), DNA damaging agents, alkylating agents, antimetabolites, spindle poison plant alkaloids, cytotoxic/antitumor antibiotics, topoisomerase inhibitors, antibodies, photosensitizers, and kinase inhibitors.

The chemotherapeutic agent may be natural or synthetic.

The chemotherapeutic agent may be a small molecule.

Chemotherapeutic agents for use for the treatment of colorectal cancer can include but are not limited to 5-fluorouracil, capecitabine, irinotecan, oxaliplatin, and a combination or trifluridine and tipiracil.

Targeted chemotherapeutic agents block specific proteins or genes. Targeted therapeutic agents include but are not limited to denosumab, romidepsin, ofatumumab, pazopanib, everlimus, nilotinib, temisirolimus, lapatinib, sunitinib, dasatinib, vorinostat, erlotinib, bevacizumab, cetuximab, bortezomib, gefitinib, ibritumomab, alemtuzumab, imatinib, gentuzumab, denileukin difitox, trastumab, rituximab, ramucirumab, ziv-aflibercept, panitumumab, and regorafenib.

Immunotherapy would include but is not limited to PD-1 inhibitors including pembrolizumab and nivolumab, and a CTLA-4 inhibitor including ipilimumab.

The methods and compositions described can be used in combination with other antineoplastic agents or immunogenic agents in order to improve their efficacy, for example, attenuated cancerous cells, tumor antigens, antigen presenting cells such as dendritic cells pulsed with tumor derived antigen or nucleic acids, immune stimulating cytokines (for example, IL-2, IFNa2, GM-CSF), and cells transfected with genes encoding immune stimulating cytokines such as but not limited to GM-CSF).

### Cancers to be Treated and/or Prevented

The methods and compositions described herein can be used to treat cancer (*i.e.,* to inhibit the growth or survival of tumor cells). As described herein, Annexin A1 has been identified as a tumor promoter independent of *Fn.* The promoting role of *Fn* in CRC is due to its stimulation of Annexin A1. Thus, this methods and compositions described can be used to treat cancers whose growth is enhanced by Annexin A1, regardless of whether *Fn* is present.

Non-limiting examples of preferred cancers for treatment and/or prevention include melanoma (*e.g.,* metastatic malignant melanoma), renal cancer (*e.g.* clear cell carcinoma), prostate cancer (*e.g.,* hormone refractory prostate adenocarcinoma), pancreatic adenocarcinoma, colon cancer or colorectal (CRC), lung cancer (*e.g*., non-small cell lung cancer), esophageal cancer, squamous cell carcinoma of the head and neck, liver cancer, ovarian cancer, cervical cancer, thyroid cancer, glioblastoma, glioma, and leukemia. Additionally, included are refractory or recurrent malignancies whose growth may be inhibited using compositions described herein.

The methods and compositions described herein can be also be used to prevent cancer in particular colorectal cancer in patients with FAP, as well as patients at risk for recurrence such as a subject who has a high level of Annexin A1 in their colorectal cancer tissue.

### The Expression of Annexin A1 as an Independent Prognosis Biomarker of CRC and Methods of Detecting Such

Also described herein is the increased expression of *ANXA-A1* mRNA in CRC tissue correlated to colon cancer recurrence independent of cancer stage, grade, age and sex (Example 8).

By using the differential expression of *ANXA1,* important predictions and determinations can be made regarding the severity and treatment of a patient's disease. While tests for this biomarker can be performed at any time after a diagnosis of CRC, preferably such tests would be performed as soon as possible after a positive diagnosis of CRC is made by a clinician. In that manner, the valuable insight into the disease can be utilized in choice of therapy.

Thus, disclosed herein is a test for the expression of *ANXA1* could be done. If the expression is increased as compared to a reference value, then the patient is identified as having a poor prognosis and more aggressive treatment should be considered.

Aggressive treatment for colorectal cancer could include the administration of an agent which inhibits or blocks Annexin A1 either alone or in combination with chemotherapeutic agents, targeted therapeutic agents and/or immunotherapy. Chemotherapeutic agents can include but are not limited to 5-fluorouracil, capecitabine, irinotecan, oxaliplatin, and a combination or trifluridine and tipiracil. Targeted therapeutic agents include but are not limited to drugs that target VEGF including bevacizumab, ramucirumab, and ziv-aflibercept, drugs that target EGFR including cetuximab and panitumumab, and regorafenib. Immunotherapy would include but is not limited to PD-1 inhibitors including pembrolizumab and nivolumab, and a CTLA-4 inhibitor including ipilimumab. The addition of the agent which inhibits or blocks Annexin A1 can reduce resistance to and/or improve the efficacy of these other therapeutic agents.

The presence or amount of the gene expressions can be compared to a reference value. Optionally, the reference value is the level of gene expression of Annexin A1 from a healthy control tissue. Optionally, the healthy control tissue is healthy colorectal tissue.

Optionally, a sample of tissue from colorectal cancer tissue from a subject with CRC is obtained.

The nucleic acid is extracted, isolated and purified from the cells of the tissue or fluid by methods known in the art.

If required, a nucleic acid sample are prepared using known techniques. For example, the sample can be treated to lyse the cells, using known lysis buffers, sonication, electroporation, with purification and amplification occurring as needed, as will be understood by those in the skilled in the art. In addition, the reactions can be accomplished in a variety of ways. Components of the reaction may be added simultaneously, or sequentially, in any order. In addition, the reaction can include a variety of other reagents which can be useful in the methods and assays and would include but is not limited to salts, buffers, neutral proteins, such albumin, and detergents, which may be used to facilitate optimal hybridization and detection, and/or reduce non-specific or background interactions. Also reagents that otherwise improve the efficiency of the assay, such as protease inhibitors, nuclease inhibitors, and anti-microbial agents, can be used, depending on the sample preparation methods and purity.

Once prepared, mRNA or other nucleic acids are analyzed by methods known to those of skill in the art. In addition, when nucleic acids are to be detected preferred methods utilize cutting or shearing techniques to cut the nucleic acid sample containing the target sequence into a size that will facilitate handling and hybridization to the target. This can be accomplished by shearing the nucleic acid through mechanical forces, such as sonication, or by cleaving the nucleic acid using restriction endonucleases, or any other methods known in the art. However, in most cases, the natural degradation that occurs during archiving results in "short" oligonucleotides. In general, the methods and assays of the invention can be done on oligonucleotides as short as 20-100 base pairs, with from 20 to 50 being preferred, and between 40 and 50, including 44, 45, 46, 47, 48 and 49 being the most preferred.

Methods for examining gene expression, are often hybridization based, and include, Southern blots; Northern blots; dot blots; primer extension; nuclease protection; subtractive hybridization and isolation of non-duplexed molecules using, for example, hydroxyapatite; solution hybridization; filter hybridization; amplification techniques such as RT-PCR and other PCR-related techniques such as PCR with melting curve analysis, and PCR with mass spectrometry; fingerprinting, such as with restriction endonucleases; and the use of structure specific endonucleases. mRNA expression can also be analyzed using mass spectrometry techniques (e.g., MALDI or SELDI), liquid chromatography, and capillary gel electrophoresis. Any additional method known in the art can be used to detect the presence or absence of the transcripts.

For a general description of these techniques, see also Sambrook *et al.* 1989; Kriegler 1990; and Ausebel *et al.* 1990.

Screening and diagnostic method may involve the amplification of the target loci. A preferred method for target amplification of nucleic acid sequences is using polymerases, in particular polymerase chain reaction (PCR). PCR or other polymerase-driven amplification methods obtain millions of copies of the relevant nucleic acid sequences which then can be used as substrates for probes or sequenced or used in other assays.

Amplification using polymerase chain reaction is particularly useful in the embodiments of the current invention. PCR is a rapid and versatile in vitro method for amplifying defined target DNA sequences present within a source of DNA. Usually, the method is designed to permit selective amplification of a specific target DNA sequence(s) within a heterogeneous collection of DNA sequences (e.g. total genomic DNA or a complex cDNA population). To permit such selective amplification, some prior DNA sequence information from the target sequences is required. This information is used to design two oligonucleotide primers (amplimers) which are specific for the target sequence and which are often about 15-25 nucleotides long.

Alternatively, Annexin A1 protein can be isolated and/or purified from a sample of colorectal cancer tissue using any method known in the art, including but not limited to immunoaffinity chromatography.

While any method known in the art can be used, preferred methods for detecting and measuring increase levels of the proteins in a protein sample include flow cytometry, quantitative Western blot, immunoblot, quantitative mass spectrometry, enzyme-linked immunosorbent assays (ELISAs), radioimmunoassays (RIA), immunoradiometric assays (IRMA), and immunoenzymatic assays (IEMA) and sandwich assays using monoclonal and polyclonal antibodies.

Antibodies are a preferred method of detecting and measuring target or desired proteins in a sample. Such antibodies are available commercially or can be made by conventional methods known in the art. Such antibodies can be monoclonal or polyclonal and fragments thereof, and immunologic binding equivalents thereof. The term "antibody" means both a homologous molecular entity as well as a mixture, such as a serum product made up of several homologous molecular entities.

Optionally, such antibodies will immunoprecipitate the desired proteins from a solution as well as react with desired/target proteins on a Western blot, immunoblot, ELISA, and other assays listed above.

Antibodies for use in these assays can be labeled covalently or non-covalently with an agent that provides a detectable signal. Any label and conjugation method known in the art can be used. Labels, include but are not limited to, enzymes, fluorescent agents, radiolabels, substrates, inhibitors, cofactors, magnetic particles, and chemiluminescent agents. A number of fluorescent materials are known and can be utilized as detectable labels. These include, for example, fluorescein, rhodamine, auramine, Texas Red, AMCA blue and Lucifer Yellow. A particular detecting material is anti-rabbit antibody prepared in goats and conjugated with fluorescein through an isothiocyanate. Any desired targets or binding partner(s) can also be labeled with a radioactive element or with an enzyme. The radioactive label can be detected by any of the currently available counting procedures. The preferred isotope may be selected from ³H, ¹⁴C, ³²P, ³⁵S, ³⁶Cl, ⁵¹Cr, ⁵⁷Co, ⁵⁸Co, ⁵⁹Fe, ⁹⁰Y, ¹²⁵I, ¹³¹I, and ¹⁸⁶Re. Enzyme labels are likewise useful, and can be detected by any of the presently utilized colorimetric, spectrophotometric, fluorospectrophotometric, amperometric or gasometric techniques. The enzyme is conjugated to the selected particle by reaction with bridging molecules such as carbodiimides, diisocyanates, glutaraldehyde and the like. Many enzymes which can be used in these procedures are known and can be utilized. The enzymes can be are peroxidase, β-glucuronidase, β-D-glucosidase, β-D-galactosidase, urease, glucose oxidase plus peroxidase and alkaline phosphatase. U.S. Patent Nos. 3,654,090; 3,850,752; and 4,016,043 are referred to by way of example for their disclosure of alternate labeling material and methods.

An alternative method for detection of the protein markers is to perform flow cytometry analysis on cells obtained from colorectal cancer tissue from the subject.

### Kits

It is contemplated that all of the methods disclosed herein can be in kit form for use by a health care provider and/or a diagnostic laboratory.

Disclosed is a kit comprising one or more probes and/or one or more antibodies for detecting expression levels of *ANXA1* as described herein.

Assays for the detection and quantitation of *ANXA1* gene expression can be incorporated into kits. Such kits may include probes for *ANXA1,* reagents for isolating and purifying nucleic acids from biological tissue or bodily fluid, reagents for performing assays on the isolated and purified nucleic acid, instructions for use, and reference values or the means for obtaining reference values in a control sample for the *ANXA1.*

These kits may have the probes attached to a solid state.

Assays for the detection and quantitation of Annexin A1 protein can be incorporated into kits. Such kits may include antibodies that recognize the peptide of interest, reagents for isolating and/or purifying protein from a biological tissue or bodily fluid, reagents for performing assays on the isolated and purified protein, instructions for use, and reference values or the means for obtaining reference values for the quantity or level of Annexin A1 in a control sample.

Commercial test kits suitable for use by a medical specialist may be prepared to determine the presence or amount of a desired gene or protein activity, expression or gene amplification in samples from colorectal cancer patients.

In accordance with the above, an assay system for screening potential drugs effective to modulate the activity or expression of *ANXA1* is disclosed. The target may be introduced into a test system, and the prospective drug may also be introduced into the resulting cell culture, and the culture thereafter examined to observe any changes in the target activity of the cells, or in the proliferation or division of the cells, due either to the addition of the prospective drug alone, or due to the effect of added quantities of the known target.

### EXAMPLES

This invention will be better understood from the Experimental Details, which follow. However, one skilled in the art will readily appreciate that the specific methods and results discussed are merely illustrative of the invention as described more fully in the claims that follow thereafter.

### Example 1 - Materials and Methods for Examples 2-7

**Bacterial strains and cell cultures.** *E. coli* DH5α was grown at 37°C in LB broth in air. Wild-type *Fn* 12230 and its *fadA*-deletion mutant US1 were grown at 37°C in Columbia broth supplemented with 5 µg/ml hemin, 1 µg/ml menadione under anaerobic condition (90% N₂, 5% CO₂, and 5% H₂). To prepare CFSE-labeled *Fn,* the bacteria were grown to mid-log phase (A₆₀₀ = 0.3) and washed twice with PBS followed by incubation in PBS containing 50 µM of 5-(and-6)-carboxyfluoroscein diacetate succinimidyl ester (CFSE; Invitrogen, Grand Island, NY) at room temperature for 30 minutes with gentle agitation. The labeled bacteria were washed ten times with PBS and re-suspended in PBS. The labeled bacteria were plated on the Tryptic soy agar supplemented with 5 µg/ml hemin, 1 µg/ml menadione, and 5% defibrinated sheep blood to numerate the viable bacterial cells. Cell cultures AA/C1, AA/C1/SB (aka SB), AA/C1/SB/10C (aka 10C), HCT116, DLD1, SW480, HT29, and MCF-7 were maintained as previously described (Rubinstein *et al.* 2013; Williams 1990).

**Plasmid construction and DNA/RNA transfections.** Full length *ANXA1* was amplified by PCR using primers listed in Table 1 and cloned in to pcDNA^{™}3.1 (+) Mammalian Expression Vector. Plasmid transfection was performed using lipofectamine 2000 (Invitrogen, CA) following the manufacturer instructions. Control siRNA, *ANXA1-*specific siRNA and *CDH1*-specific siRNA were purchased from Invitrogen (CA). The siRNA utilized in these experiments included: Negative control: Invitrogen Stealth RNAi siRNA negative control cat number 12935300; Annexin siRNA: Invitrogen stealth siRNA (cat # ANXA1HSS100502) sequence UAA CCA UUA UGG CCU UAU GCA AGG C (SEQ ID NO: 1); and E-cadherin siRNA: Invitrogen stealth siRNA (cat # CDH1HSS101669) sequence UUA AUC UCC AGC CAG UUG GCA GUG U (SEQ ID NO: 2). Lipofectamine RNAi MAX (Invitrogen, CA) was used for the siRNA transfection following manufacturer instructions.

**Antibodies.** Annexin antibodies utilized in these experiments included: Annexin A1 (Thermo Fisher Scientific, cat # 71-3400); Annexin A2 (Thermo Fisher Scientific, cat # PA5-27566); Annexin V (Thermo Fisher Scientific, cat # PA5-27872); Annexin A6 (Thermo Fisher Scientific, cat. # 720161); and Annexin A11 (Thermo Fisher Scientific, cat # PA5-68093).

**Protein purification and conjugation.** FadAc and mFadA were purified as previously described (Xu *et al.* 2007). To conjugate the proteins, 3 mg each of FadAc, mFadA, and BSA (MP Biomedicals, Santa Ana, CA) were mixed with 10 mg/ml of Alexa Fluor^{™} 488 tetrafluorophenyl (TFP) ester (Invitrogen, CA) and vortexed at room temperature for 1 hour. Following the reaction, unconjugated Alexa Fluor^{™}488 TFP was removed using the PD-10 Desalting column (GE Healthcare Life Sciences, Buckinghamshire, UK). The amount of labeled protein was quantified using a spectrophotometer (NanoDrop Technologies, Wilmington, DE).

**Cell proliferation assay.** Cells were seeded in 24 well plates at 5 x 10⁴ cells per well. Cells were untreated or incubated with bacteria at an MOI of 1000:1. Cell numbers were counted at indicated time points using a hemocytometer as previously described (Rubinstein *et al.* 2013). Each experiment was performed in triplicate and repeated at least three times.

**Cell culture attachment and invasion assay.** The assay was performed as previously described (Han *et al.* 2000). Briefly, host cells were seeded in 24 well plates and grown until 80% confluency. Bacteria were added at an MOI 50:1 and incubated for 1 hour at 37°C in 5% CO₂. Following washes with PBS and lysis with water for 20 minutes, serial dilutions of the lysates were plated onto blood agar plates to enumerate the viable bacterial counts. For the invasion assay, bacteria were incubated with the host cells for 3 hours followed by treatment with 300 µg/ml gentamicin and 200 µg/ml metronidazole for 1 hour at 37°C. Following washes with PBS, the cells were lysed with water and intracellular bacterial counts were determined as described above. The levels of attachment and invasion were expressed as the percentage of bacteria recovered following cell lysis relative to the total number of bacteria initially added. Each experiment was performed in triplicate and repeated at least three times.

**Flow cytometry.** Approximately a total of 2 x 10⁵ cells were incubated with *Fn* or purified FadA in 35 mm dish at 37°C for indicated time periods. After washing with PBS, the cells were incubated with enzyme-free cell dissociation buffer (Thermo Fisher Scientific) for 5 min at 37°C followed by addition of 1 ml DMEM. The cells were collected and centrifuged at 500 g for 3 minutes. The cell pellet was fixed with 75% cold ethanol and kept at -20°C. The pellet was centrifuged again at 2500 g for 10 minutes and washed with phosphate citrate buffer (200 mM Na₂HPO₄, 100 mM citric acid, pH 7.4), followed by blocking with PBS containing 2% skim milk at room temperature for 1 hour. After washing with PBS, the cells were incubated with rabbit anti-AnnexinA1 polyclonal IgG (1:400 dilution, Invitrogen), or rabbit anti-β-catenin polyclonal IgG (1:200 dilution, Invitrogen), or rabbit IgG isotype control (Invitrogen) at room temperature for 1 hour. The cells were then washed with PBS and incubated with Alexa Fluor^{®}700-conjugated goat anti-rabbit IgG (1:1000 dilution, Invitrogen) for 1 hour. After washing the cells with PBS, the flow cytometric data were acquired by a BD LSR II flow cytometer and analyzed using Flow Jo software (Tree Star, San Carlos, CA).

**Immunofluorescent staining.** An aliquot of 1x10³-1x10⁵ cells were seeded into Nunc Lab-Tek II Chamber Slide System (Thermo Fisher Scientific) in 400 µl and allowed to grow for 2-5 days till reaching desired confluency. Following washes with DMEM, the cells were incubated with CFSE-labeled *Fn* or Alexa Fluor^{™}488-conjugated FadAc, mFadA, and BSA (300 µg/ml) for indicated time periods. Following washes of ten times with DMEM, the cells were fixed in PBS containing 4% paraformaldehyde at room temperature for 15 minutes, followed by neutralization in PBS containing 1% glycine at room temperature for 15 minutes. After blocking with PBS containing 2% skim milk and 0.3% Triton X-100 for 1 hour at 4°C, the cells were incubated overnight at 4°C with goat anti-human E-cadherin polyclonal antibodies (1:400 dilution, R&D Systems, MN) and rabbit anti-AnnexinA1 polyclonal antibodies (1:400 dilution, Invitrogen) or rabbit anti-β-catenin polyclonal IgG (1:400 dilution, Invitrogen) in blocking solution. After washing three times with PBS containing 0.3% Triton X-100, the cells were incubated with Cy3-conjugated donkey anti-goat IgG (1:1000 dilution, Jackson ImmunoResearch, West Grove, PA) and Alexa Fluor^{®}680-conjugated donkey anti-rabbit IgG (1:1000 dilution, Invitrogen), washed, and covered in mounting medium containing DAPI (Vector Laboratories, CA, USA). The samples were visualized with a Nikon Ti Eclipse inverted microscope for scanning confocal microscopy.

For immunofluorescent staining of human colonic specimens, frozen sections of the paired tumor and normal tissues were obtained. The slides were fixed in 4% paraformaldehyde for 15 minutes, and permeabilized using 0.1% Triton x-100 in PBS followed by blocking of nonspecific binding. The slides were then incubated with rabbit anti-AnnexinA1 polyclonal antibodies (Thermo Fisher Scientific) and mouse anti-FadA monoclonal antibody 5G11 (Xu *et al.* 2007), or with isotype controls, *i.e.,* rabbit IgG (Invitrogen) and mouse IgG (R&D Systems, Minneapolis, MN). After washes, the slides were incubated with Alexa Fluor^{®}680-conjugated donkey anti-rabbit (Invitrogen) and Alexa Fluor^{®}555-conjugated goat anti-mouse (Invitrogen), washed, and covered in mounting medium containing DAPI (Vector Laboratories). The slides were visualized with a Nikon Ti Eclipse inverted microscope for scanning confocal microscopy.

**Western-blot analysis.** RKO cells transfected with *ANXA1* expression vector or expression vector alone were seeded in 6-well plates and grown for 2 days. The cells were washed with ice-cold PBS and lysed with RIPA lysis buffer (EMD Millipore, Burlington, MA) containing HaltTM Protease & Phosphatase Inhibitor Single-Use Cocktail (Thermo Fisher Scientific, MA). The cell lysates were centrifuged at 13,000 x g for 10 minutes at 4°C and the protein concentration was measured using the BCA protein assay kit (Thermo Fisher Scientific) according to manufacturer's instructions. One microgram of total proteins was separated by NuPAGETM 4- 12% Bis-Tris Gel (Thermo Fisher Scientific) and transferred onto PVDF membranes (Bio-Rad, Hercules, CA). The membrane was blocked with 5% skim milk in TBS containing 0.1% Tween 20 (TBST) at room temperature for 1 hour followed by incubation with antibodies against Annexin A1 (Thermo Fisher Scientific, 71-3400, 1:4000 dilution), Cyclin D1 (Thermo Fisher, Cat No 701421, 1:250 dilution), β-catenin (Thermo Fisher Scientific, 71-2700, 1:2000 dilution), or β-actin (Abcam, ab6276, 1:4000 dilution) in 0.5% skim milk in TBST at 4°C overnight. After washing three times with TBST, the membrane was incubated with HRP-conjugated secondary antibody in TBST at room temperature for 1 hour. Following washes, the immune-reactive bands were detected with ECL Western Blotting Substrate (Thermo Fisher Scientific).

**Co-immunoprecipitation.** DLD1 cells were incubated with 1 mg/ml of FadAc for 0, 15, and 120 minutes, respectively. Rabbit anti-Annexin A1 antibody (Thermo Fisher Scientific, MA), mouse anti-FadA antibody 5G11 (Xu *et al.* 2007), rabbit IgG or mouse IgG were bound covalently to the agarose resins using the Pierce Co-Immunoprecipitation Kit (Thermo Fisher Scientific) following the manufacturer's instructions. The cells were lysed and mixed with the antibody-coupled resins and incubated for 2 hours at room temperature. Following washes, the complex bound to the antibodies was eluted and examined by Western-blot analysis as described above. Following electrophoresis and transfer, the PVDF membranes were incubated with rabbit anti-AnnexinA1 polyclonal antibodies (Thermo Fisher Scientific,), mouse anti-FadA monoclonal antibodies 7H7, rabbit anti-E-cadherin monoclonal antibodies (Cell Signaling Technology), mouse anti-β-actin monoclonal antibodies (Cell Signaling Technology) or rabbit anti-β-catenin polyclonal antibodies (Thermo Fisher Scientific). After washes, the membranes were incubated with HRP-conjugated goat anti-rabbit IgG antibodies (Bio-Rad) or PolyHRP-conjugated goat anti-mouse IgG antibodies (Thermo Fisher Scientific). The membranes were washed and incubated with SuperSignal West Pico Chemiluminescent Substrate (Thermo Fisher Scientific) and the bands were detected using Chemidoc MP Imaging System (Bio-Rad).

***APC*^{*min*/*+*} mouse model.** *APC*^{*min*/*+*} mice were obtained from Jackson Laboratories. All mice were kept in sterilized filtered-topped cages in a room with 12-hr light cycle, fed autoclaved food and water ad libitum, and handled in a laminar flow hood. Prior to bacterial inoculation, the mice were provided with antibiotics-supplemented drinking water (1 g/L ampicillin and 1 g/L metronidazole) for 2 weeks. Bacteria cultures were grown and pelleted at speed of 5000 g for 5 minutes and re-suspended in PBS to an estimated density of 2 x 10¹⁰ CFU/ml. An aliquot of 50 µl of the bacterial suspension was gavaged at 3 times a week for 8 weeks. At the end of the treatment, animals were sacrificied and the colons were collected, washed with PBS and opened longitudinally. Tumors were counted. Tumors and normal intestinal tissues were collected for histological analysis and extraction of DNA and RNA.

**Tumor xenografts.** Four-week old nude mice were purchased from Taconic Biosciences (NY, USA). An inoculum of 1x10⁷ HCT 116 cells treated with either control or *ANXA1*-specific siRNA were injected subcutaneously and bilaterally into the nude mice, with control cells injected on the left side and *ANXA1*-knockdown cells on the right side. On day 7-9 post-injection, the tumor length and width were measured using calipers and tumor volumes were calculated using the following formula: Volume=(width)² × length/2.

**Clinical specimens.** A total of 18 CRC cases were retrieved from files at the Department of Pathology at Columbia University Medical Center. Hematoxylin and eosin (H&E) slides were reviewed to confirm the presence of colorectal adenocarcinoma. Frozen sections of the tumors and paired normal tissues were used for DNA/RNA extraction and immunofluorescent staining.

**DNA and RNA extraction and real-time quantitative PCR.** RNA was extracted from cultured cells using QIAGEN RNeasy Mini Kit (Germany) following manufacturer's instructions. All Prep DNA/RNA Mini Kit (Germany) was used to extract DNA and RNA from normal and tumor tissues from mice and clinical specimens. Lysis buffer and 0.1 mm glass beads (Mo Bio Laboratories, Carlsbed, CA) were added to the samples, followed by homogenization for 60 seconds in FastPrep-24 (MP Biomedicals). DNA and RNA concentrations were measured δδδusing NanoDrop ND 1000 spectrophotometer (NanoDrop Technologies, DE). For DNA, samples were diluted to 30 ng/µl and 1 µl was used for real-time qPCR. For RNA, reverse transcription was performed using Superscript IV First-Strand Synthesis System (Invitrogen) following manufacturer's instructions. Real-time qPCR was performed in StepOnePlus (Applied Biosystems, CA) in duplicates using primers listed in Table 1. To quantify gene copies, standard curves using plasmids carrying 16S rRNA gene or *fadA* gene were generated. For RNA, data were analyzed using the 2(-ΔΔC(T)) method (Livak *et al.* 2001) and normalized to the β-actin control.

**Statistical analysis.** The differences between groups were examined by two-tailed t-test, one-way or two-way ANOVA followed by Student-Newman-Keuls (SNK) tests. For the clinical specimens, the Kruskal-Wallis nonparametric test was performed, followed by the Conover test. *p<* 0.05 was considered statistically significant.

**Table 1- Primers used in this study**

| Primers | Sequence 5'-3' |
|---|---|
| h *ANXA1* F | GCAGGCCTGGTTTATTGAAA (SEQ ID NO: 3) |
| h *ANXA1* R | GCTGTGCATTGTTTCGCTTA (SEQ ID NO: 4) |
| *fadA* F | TAGCACAAAATGAACAAGTTTAC (SEQ ID NO: 5) |
| *fadA* R | ATAAAATCTTGTGTTAGCTTC (SEQ ID NO: 6) |
| 16S F | ACTCCTACGGGAGGCAGCAG (SEQ ID NO: 7) |
| 16S R | ATTACCGCGGCTGCTGG (SEQ ID NO: 8) |
| m *ANXA1* F | CAACCATCATTGACATTCTTACCAA (SEQ ID NO: 9) |
| m *ANXA1* R | TGGCACCACGGAGTTCATC (SEQ ID NO: 10) |
| *IL1b* F | AAACAGATGAAGTGCTCCTTCCAGG (SEQ ID NO: 11) |
| *IL1b* R | TGGAGAACACCACTTGTTGCTCCA (SEQ ID NO: 12) |
| *NFkB2* F | GGAGCAAGAGGCCAAAGAACT (SEQ ID NO: 13) |
| *NFkB2* R | TACAGGCCGCTCAATCTTCAT (SEQ ID NO: 14) |
| *RANTES* F | GCTGTCATCCTCATTGCTACTG (SEQ ID NO: 15) |
| *RANTES* R | TGGTGTAGAAATACTCCTTGATGTG (SEQ ID NO: 16) |
| *CCL20* F | CTGGCTGCTTTGATGTCAGT (SEQ ID NO: 17) |
| *CCL20* R | CGTGTGAAGCCCACAATAAA (SEQ ID NO: 18) |
| *CCND1* F | ACAAACTGTTTTGAAAATCCA (SEQ ID NO: 19) |
| *CCND1* R | CGAGTCATTGCATACTGTCC (SEQ ID NO: 20) |
| *VIL1* F | AGCCAGATCACTGCTGAGGT (SEQ ID NO: 21) |
| *VIL1* R | TGGACAGGTGTTCCTCCTTC (SEQ ID NO: 22) |
| *CDH1* F | AATCCAAAGCCTCAGGTCATAAACA (SEQ ID NO: 23) |
| *CDH1* R | GGTTGGGTCGTTGTACTGAATGGT (SEQ ID NO: 24) |
| *ANXA1* (full) F | TCAAAAATGGCAATGGTATCAGAAT (SEQ ID NO: 25) |
| *ANXA1* (full) R | GTTTCCTCCACAAAGAGCCA (SEQ ID NO: 26) |

### Example 2- F. nucleatum preferentially binds, invades and stimulates the growth of cancerous colorectal cells via Annexin A1, and Annexin A1 is selectively expressed in proliferating cancerous colorectal cells, is a novel CRC growth factor, and can be inhibited by siRNA

It has been previously shown that FadA was unable to promote growth of non-cancerous HEK293 cells even though E-cadherin was present (Rubinstein *et al.* 2013).

In order to determine the specificity of *F*. *nucleatum-*mediated growth stimulation, the effects of *F. nucleatum* strain 12230 (*Fn* 12230) was tested on the PC-9 lung cancer cells, 22RV1 prostate cancer cells, and MCF7 breast cancer cells, all of which expresses E-cadherin, as well as UMUC3 bladder cancer cells, which does not express E-cadherin. No growth stimulation was detected; on the contrary, *F*. *nucleatum* inhibited the proliferation of PC-9, 22RV1 and UMUC3 cells, presumably due to toxic effects (Fig. 1A). Therefore, it is likely that additional components specific to CRC are required for FadA to promote growth.

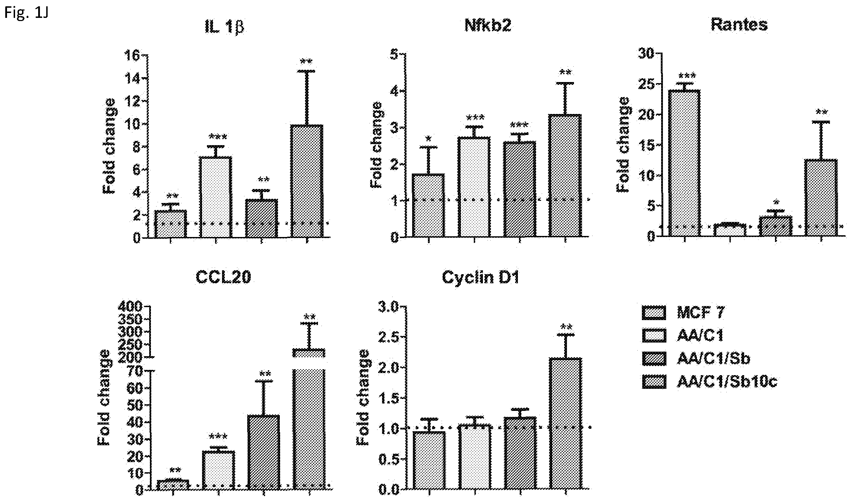

To identify possible CRC component(s), a CRC progression model was used consisting of a series of cell lines sequentially derived from a non-malignant human colonic adenoma (Williams 1990). AA/C1 is a slow growing non-tumorigenic adenoma cell line with low colony-forming efficiency. Following treatment with 1 mM sodium butyrate, it gave rise to the AA/C1/SB cell line, which grew faster with increased colony-forming efficiency but remained non-tumorigenic in mice. The AA/C1/SB cells were further mutagenized with N-methyl-N'-nitro-N-nitrosoguanidine to produce a tumorigenic cell line, AA/C1/SB/10C (Williams 1990). *Fn* 12230 accelerated the growth of the tumorigenic AA/C1/SB/10C (referred to as "10C"), but not the non-tumorigenic AA/C1 or AA/C1/SB (referred to as "SB") (Fig. 1A). Growth stimulation required FadA, as the *fad*A-deletion mutant US1 was defective. Consistent with these findings, although all the cell lines treated with *Fn* 12230 expressed increased levels of proinflammatory markers, only the 10C cells exhibited elevated expression of the oncogene Cyclin D1 (Fig. 1J). *Fn* 12230 bound 75% more, and invaded 150% more, efficiently to the tumorigenic 10C than its non-tumorigenic parent SB (Fig. 1B). These results were consistent with the inventor's previous finding that the FadA gene levels (and *Fn*) were significantly higher in the human colorectal carcinoma tissues than in the adenoma tissues (Rubinstein *et al.* 2013). The results also suggested 10C and SB may differ in their membrane components, which might explain the differential binding by *Fn.*

Comparative proteomic analysis revealed two membrane proteins that were increased in 10C compared to SB, *i.e.* Annexin A1 (*ANAX1*) and villin (Roth *et al.* 2010). Downregulation of Annexin A1 in 10C by siRNA effectively reduced Fn binding and invasion, in a similar manner as suppression of CDH1 (Fig. 1B), whereas knocking down of villin had no effect (Fig. 1C). Transfection of *ANXA1* into SB cells significantly increased *Fn* binding and invasion (Fig. 1B). These results suggested that Annexin A1 plays an important role in *Fn* interaction with the tumorigenic cell, 10C.

Annexin A1 was found to be selectively expressed in proliferating tumorigenic and CRC cells. In 10C and human CRC cells HCT116, DLD1 and RKO, ANXA1 gene expression was significantly higher in non-confluent than confluent state, while no difference of expression was observed in the non-tumorigenic SB (Fig. 1D). Among the CRC cell lines, RKO expressed significantly less *ANXA1* than others. Immunofluorescence staining revealed that Annexin A1 was expressed on the outer layer of the growing mass of tumorigenic 10C cells (Fig. 1E). In contrast, neither cell density-dependent, nor spatial expression, was observed in the non-tumorigenic SB cells.

Down regulation of Annexin A1 by siRNA inhibited the growth of 10C, HCT116, DLD1, SW480 and HT29, without affecting the non-tumorigenic SB, or the human CRC cell line RKO, which expressed the least Annexin A1 (Figs. 1D and 1F). Transfection of *ANXA1* into SB and RKO significantly stimulated their proliferation (Fig. 1G). When equal numbers of HCT116 cells treated with control or *ANXA1*-specific siRNA were subcutaneously and bilaterally injected into nude mice, suppression of *ANXA1* significantly attenuated tumor growth compared to controls (Fig. 1H). Similar results were obtained with DLD1 (Fig. 1I).

As no *Fn* was included in these experiments (Figs. 1D-1H), the results demonstrate that Annexin A1 is a critical growth factor for CRC regardless of *Fn* and can be inhibited by siRNA.

### Example 3 - F. nucleatum selectively binds to Annexin A1-expressing cells and induces further Annexin A1 expression via FadA in a positive feedback loop

When *Fn* was incubated with 10C, it immediately exhibited preferential binding to Annexin A1-expressing cells, starting at as early as 5 minutes following incubation, whereas no binding preference was detected in SB (Fig. 2A, compare solid and clear bars). Annexin A1 expression increased with continuous *Fn* incubation, with significant induction at 60 minutes, which persisted through 120 min (Fig. 2A). Such induction required FadA, as the *fad*A-deletion mutant US1 was defective, and purified FadAc induced Annexin A1 in a dose-dependent manner (Fig. 2B).

Of note, similar results were obtained upon re-analysis of a recently published, publicly available RNA-seq dataset (Yu *et al.* 2017) that contains gene-expression data from human HT29 CRC cells incubated with a different strain, *F*. *nucleatum* ATCC 25586 (Fig. 2E). Similar observations were made with HCT116 and DLD1 cells (Fig. 2F). The induction appeared to be at the transcriptional level as shown by the real-time qPCR results (Fig. 2C). The kinetics of transcriptional activation corroborated with the percentage increase of Annexin A1-positive cells and binding of Fn (compare Fig. 2C with Figs. 2A and 2F). These results suggested a positive feedback loop in which *Fn* converts Annexin A1-negative cells into Annexin A1-positive cells which in turn enhances its own binding. Consistent with the observations in Fig. 1D, *Fn* induced Annexin A1 at the outer layer of the cell mass (Fig. 2D). No induction of Annexin A1 was detected in the non-tumorigenic SB cells (Figs. 2A, 2C, 2D), suggesting the induction is specific for tumorigenic and CRC cells. The *fad*A-deletion mutant US1 did not induce Annexin A1 expression. However, it also exhibited preferential binding to Annexin A1-positive cells (Figs 2A, 2F), indicating Annexin A1 may mediate additional *F*. *nucleatum* component(s) to bind to the cancer cells.

### Example 4- FadA, E-cadherin, Annexin A1 and β-catenin form a complex in cancerous cells

FadA was previously shown to be bound to E-cadherin on CRC cells (Rubinstein *et al.* 2013). Therefore, the interactions between FadA, E-cadherin and Annexin A1 were examined. Induction of Annexin A1 expression by FadA was mediated through E-cadherin (Fig. 3A), although *Fn* did not affect E-cadherin expression at the transcription level as determined by real-time qPCR (Fig. 3I). Analysis by confocal microscopy revealed that *Fn* and FadAc co-localized with E-cadherin and Annexin A1 in 10C and DLD1 cells on cell membrane as well as intracellular (Figs. 3B and 3C). This is consistent with a previous report that binding by FadAc caused cadherins (vascular endothelial cadherin and E-cadherin) to internalize (Rubinstein *et al.* 2013). In FadAc-treated 10C cells, not only did Annexin A1 expression increase, so did co-localization of E-cadherin and Annexin A1, compared to mFadA- or BSA-treated cells (Fig. 3C). Western blot analysis demonstrated synchronized increase of E-cadherin, Annexin A1, and β-catenin, following FadAc incubation (Fig. 3D), suggesting activation of β-catenin may involve the multi-component complex. Indeed, all four components could be co-immunoprecipitated (Figs. 3E, 3F). Knocking down of Annexin A1 by siRNA abolished β-catenin activation and its nucleus translocation in 10C and DLD1 cells (Figs. 3G-3J). Thus, Annexin A1 is a necessary component of the Annexin A1-E-Cadherin-Fn complex that activates Wnt/β-catenin signaling, and in FadA-mediated tumorigenic responses.

The inventor also has reported previously that binding of *F*. *nucleatum* to E-cadherin on CRC cells activates β-catenin signaling leading to overexpression of oncogenes such as Cyclin D1 (CCND1) (Roth *et al.* 2010). When *ANXA1* was knocked down by siRNA, *F*. *nucleatum-*mediated activation of β-catenin expression was abolished in the cancerous 10C, HCT116 and DLD1 cells (Fig. 3G). Nuclear translocation of β-catenin in 10C was also inhibited (Fig. 3H). These data suggested Annexin A1 is required for activation of Wnt/ β-catenin signaling.

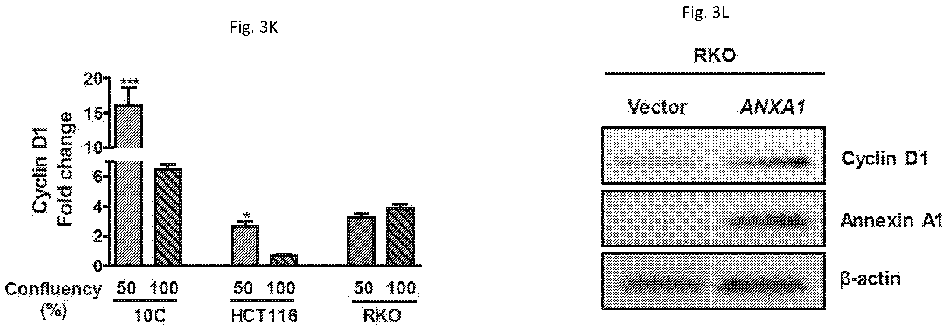

In 10C and HCT116 cells, Cyclin D1 gene expression also exhibited cell-density dependence, significantly higher in the non-confluent than confluent state, consistent with *ANXA1* expression (compare Figs. 1D and 3K). In contrast, no expression difference was detected in the RKO cells where *ANXA1* was undetectable (compare Figs. 1D and 3K). However, when *ANXA1* was transfected into the RKO cells, increased Cyclin D1 expression was observed, indicating a driving role of Annexin A1 in oncogene expression (Fig. 3L). These observations supported the role of Annexin A1 in modulating β-catenin signaling.

### Example 5- FadA and Annexin A1 co-express in colorectal tumors in mice and humans

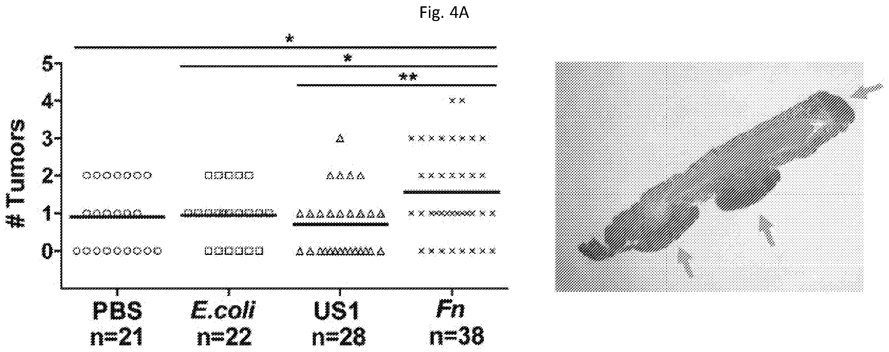

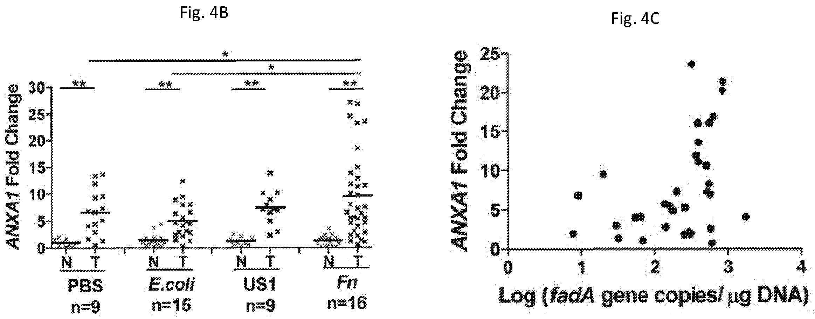

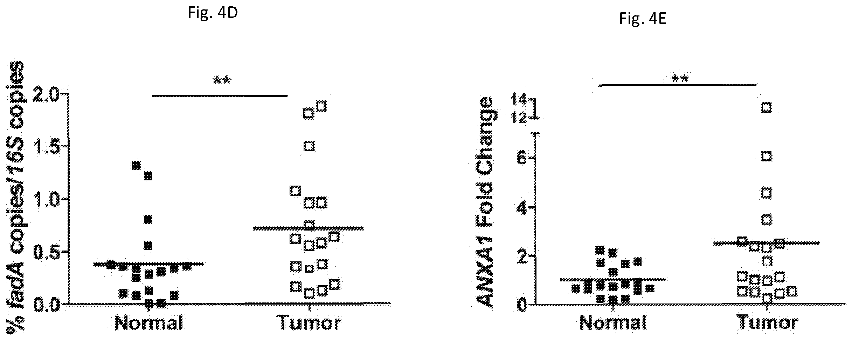

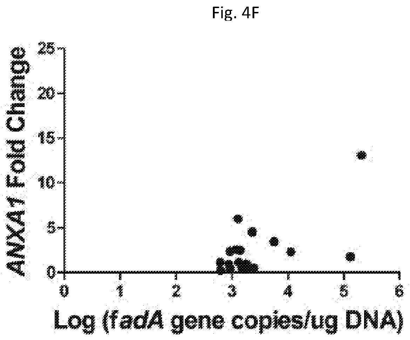

The correlation between FadA and Annexin A1 was examined *in vivo* by utilizing *APC*^{*min*/*+*} mice, which carry a mutation in one copy of the tumor suppressor gene APC and develop spontaneous tumors in the small intestine and colon. C57BL/6 *APC*^{*min*/*+*} mice gavaged with wild-type *Fn* 12230 developed significantly more tumors in the colon than those treated with the *fadA*-deletion mutant US1, *E. coli* DH5α, or PBS (Fig. 4A), demonstrating a driver role of FadA in tumorigenesis. In all treatment groups, significantly higher levels of *ANXA1* mRNA were detected in the tumors compared to the normal colonic tissues from the same mice, with the highest levels observed in those treated with wild-type *Fn* (Fig. 4B). A positive correlation between *fadA* and *ANXA1* was detected in Fn-induced tumors, with a correlation coefficient of 0.43 (p=0.01; Fig 4C). Among CRC patients, higher levels of *fadA* and *ANXA1* were detected in the adenocarcinoma tissues than the adjacent normal tissues (Figs. 4D, 4E), with correlation coefficient of 0.62 (p<0.001; Fig 4F). Immunofluorescent staining of paired tumor and normal tissues confirmed this finding, with significantly higher levels of FadA and Annexin A1 proteins detected in the tumors. Co-localization of FadA and Annexin A1 was observed in the tumors but not in the normal tissues (Fig. 4G). These results further supported the role of FadA and Annexin A1 in tumorigenesis.

### Example 6- Annexin A1 can be inhibited by anti-Annexin A1 antibodies, which in turn inhibits the growth of cancer cells in vitro and in vivo

Similar to the results shown for siRNA in Example 2 and Figs. 1F, 1H, and 1I, downregulation of Annexin A1 by an Annexin A1 antibody, suppressed tumor growth both *in vitro* and *in vivo.*

Down regulation of Annexin A1 by anti-Annexin A1 antibodies inhibited the growth of HCT116. However, antibodies to Annexin A2, Annexin V, Annexin A6 and Annexin A11 had not effect on the growth of the cells (Fig. 5A).

An aliquot of 2-8x10⁶ human CRC cells HCT 116 were inoculated into nude mice bilaterally. After 3 days, the tumors were injected either daily with 5 ul of antibodies or every other day with 10 ul of antibodies. The tumor length and width were measured using calipers and the tumor volumes were calculated using the following formula: Volume = width2 x length/2. Suppression of Annexin A1 significantly attenuated tumor growth compared to controls, in both treatment protocols (Fig. 5B). The tumors treated with the anti-Annexin A1 antibody showed no Annexin A1 in the tumor tissue (Fig. 5C).

### Example 7- Eliminating Annexin A1 in a mouse model of familial adenomatous polyposis (FAP) significantly prolonged life

*Anxa1*^{*-*/*-*} mice were obtained from Dr. Mauro Perretti at Queen Mary University (London, UK). These mice were bred with *Apc*^{*min*/*+*} mice (Jackson Laboratory) which carry a mutation in one copy of the tumor suppressor gene APC and develop spontaneous tumors in the small intestine and colon to generate *Anxa1*^{*+*/}*⁻Apc*^{*min*/*+*} and *Anxa1*^{*-*/}*⁻Apc*^{*min*/*+*} cohorts.

Eliminating Annexin A1 in the APC mutant mice prolonged their lives in a dose dependent manner, with the *ANXA1*^{*-*/*-*} APC mutant mice living significantly longer than *ANXA1*^{*+*/*+*} APC mutant mice (Fig. 6).

### Example 8- Annexin A1 is a novel colon cancer prognosis marker

The relationship between ANXA1 mRNA expression levels and risk of recurrence in CRC was investigated in a database of 466 primary colon carcinomas, obtained by assembling four independent gene-expression array datasets downloaded from the NCBI-GEO online repository (GSE14333, GSE17538, GSE31595, GSE37892), as previously described (Dalerba *et al.* 2016). The association was first tested using Kaplan-Meier survival curves, using three different approaches for patient stratification: (1) based on the median of ANXA1 mRNA expression levels (Fig. 7A); (2) based on the quartile distribution of *ANXA1* mRNA expression levels (Fig. 7B); and (3) based on expression thresholds calculated using the StepMiner algorithm (Fig. 7C), as previously described (Dalerba *et al.* 2011; Sahoo *et al.* 2007). The association between *ANXA1* mRNA expression levels and risk of recurrence was tested using both univariate and multivariate analyses based on the Cox proportional hazards method, where *ANXA1* mRNA expression levels were modeled as a continuous variable.

High levels of ANXA1 mRNA expression were associated with a statistically significant reduction in disease-free survival (DFS) rates and signficant increase in risk of recurrence, irrespective of the method used for the stratification (p<0.001, log-rank test). Differences in *ANXA1* mRNA expression levels did not appear to correlate with differences in each tumor's relative content of epithelial cells (*i.e.* tumor cell density) as revealed by the lack of visual correlations with the epithelial cell marker Desmoplakin (DSP). Importantly, the association between high levels of *ANXA1* mRNA expression and increased risk of recurrence remained statistically significant in a series of multivariate analyses (Cox proportional hazards method) that excluded clinical stage, pathological grade, age and sex as possible confounding variables, even after modeling *ANXA1* expression as a continuous variable and both when tested across the whole database (n=466; p<0.001) (Table 2) and when tested in the subset of patients annotated for the pathological grading of the respective tumors (n=216; p<0.001) (Table 3).

The hazard ratio (HR) for disease recurrence associated with increased *ANXA1* expression levels was 1.44 (95% CI 1.24-1.68; p<0.001) when tested alongside clinical stage, age and sex across the full patient cohort (n=466), and 1.56 (95% CI 1.21-2.02; p<0.001) when tested alongside clinical stage, pathological grade, age and sex in the patient subgroup annotated with pathological grade (n=216).

**Table 2- Relationship between ANXA1 mRNA expression levels and risk of recurrence in colon cancer patients- all (n=466)**

| **Cox proportional hazards model** | | | | | | | | | |
|---|---|---|---|---|---|---|---|---|---|
| **Univariate** | | | | | | **Multivariate** | | | |
| | **HR¹** | **95%Cl²** | **p-value** | | **HR¹** | | **95%Cl²** | **p-value** | |
| **ANXA1³** | 1.46 | 1.25-1.71 | <0.001 | *** | 1.44 | | 1.24-1.68 | <0.001 | *** |
| **Stage (I-IV)** | 3.47 | 2.62-4.59 | <0.001 | *** | 3.73 | | 2.74-5.09 | <0.001 | *** |
| **Age³** | 0.99 | 0.97-1.00 | 0.06 | | 1.00 | | 0.98-1.01 | 0.75 | |
| **Sex (M/F)⁴** | 1.07 | 0.89-1.28 | 0.49 | | 1.05 | | 0.88-1.27 | 0.58 | |

| | | | | | | | | | |
|---|---|---|---|---|---|---|---|---|---|
| 1 HR: hazard ratio 2 CI: confidence interval 3 ANXA1 mRNA levels and age modeled as a continuous variable 4 M/F: male vs. female *** p < 0.001 | | | | | | | | | |

**Table 3 - Relationship between ANXA1 mRNA expression levels and risk of recurrence in colon cancer patients annotated with information on tumor grade (n = 216)**

| **Cox proportional hazards model** | | | | | | | | |
|---|---|---|---|---|---|---|---|---|
| | | | | | | | | |

| **Univariate** | | | | | **Multivariate** | | | |
|---|---|---|---|---|---|---|---|---|
| | | | | | | | | |

| | **HR¹** | **95%Cl²** | **p-value** | | **HR¹** | **95%Cl²** | **p-value** | |
|---|---|---|---|---|---|---|---|---|
| **ANXA1³** | 1.48 | 1.18-1.87 | <0.001 | *** | 1.56 | 1.21-2.02 | <0.001 | *** |
| **Stage (I-IV)** | 3.13 | 2.14-4.60 | <0.001 | *** | 3.63 | 2.31-5.70 | <0.001 | *** |
| **Grade (G1-G3)** | 1.63 | 0.94-2.82 | 0.08 | | 1.20 | 0.58-1.79 | 0.95 | |
| **Age³** | 0.99 | 0.97-1.00 | 0.2 | | 1.00 | 0.98-1.02 | 0.85 | |
| **Sex (M/F)⁴** | 1.15 | 0.88-1.51 | 0.32 | | 1.16 | 0.86-1.55 | 0.33 | |

| | | | | | | | | |
|---|---|---|---|---|---|---|---|---|
| 1 HR: hazard ratio 2 CI: confidence interval 3 ANXA1 mRNA levels and age modeled as a continuous variable 4 M/F: male vs. female *** p < 0.001 | | | | | | | | |

### References

ACS (2012) Cancer Facts & Figures 2012. In pp 1-66. American Cancer Society (ACS)
Boudhraa et al., Annexin A1 localization and its relevance to cancer. Clinical science 130: 205-20 (2016).
Bullman et al., Analysis of Fusobacterium persistence and antibiotic response in colorectal cancer. Science 358: 1443-1448 (2017).
Dalerba et al., CDX2 as a Prognostic Biomarker in Stage II and Stage III Colon Cancer. N Engl J Med 374: 211-22 (2016).
Dalerba et al., Single-cell dissection of transcriptional heterogeneity in human colon tumors. Nature biotechnology 29: 1120-7 (2011).
Dulal and Keku, Gut microbiome and colorectal adenomas. Cancer journal 20: 225-31 (2014).
Fardini et al., Fusobacterium nucleatum adhesin FadA binds vascular endothelial cadherin and alters endothelial integrity. Mol Microbiol 82: 1468-1480 (2011).
Fearon and Vogelstein, A genetic model for colorectal tumorigenesis Cell 61: 759-67 (1990) Guo et al., Potential role of Anxa1 in cancer. Future oncology 9:1773-93 (2013).
Han et al., Interactions between periodontal bacteria and human oral epithelial cells: Fusobacterium nucleatum adheres to and invades epithelial cells. Infect Immun 68: 3140-3146 (2000).
Hussan et al., Fusobacterium's link to colorectal neoplasia sequenced: A systematic review and future insights. World journal of gastroenterology 23: 8626-8650 (2017).
Keku et al., The gastrointestinal microbiota and colorectal cancer. American journal of physiology. Gastrointestinal and liver physiology 308: G351-63 (2015).
Kostic et al., Fusobacterium nucleatum potentiates intestinal tumorigenesis and modulates the tumor-Immune microenvironment. Cell Host Microbe 14: 207-215 (2013).
Livak et al., Analysis of relative gene expression data using real-time quantitative PCR and the 2(-Delta Delta C(T)) Method. Methods 25: 402-408 (2001).
Luthra et al., Micro-RNA-196a targets annexin A1: a microRNA-mediated mechanism of annexin A1 downregulation in cancers. Oncogene 27: 6667-78 (2008).
Mima et al., Fusobacterium nucleatum in Colorectal Carcinoma Tissue According to Tumor Location. Clinical and translational gastroenterology 7: e200 (2016).
Mima et al., Fusobacterium nucleatum in colorectal carcinoma tissue and patient prognosis. Gut 65: 1973-1980 (2016).
Onozawa et al., Annexin A1 is involved in resistance to 5-FU in colon cancer cells. Oncology reports 37: 235-240 (2017).
Perretti et al., Annexin A1 and glucocorticoids as effectors of the resolution of inflammation. Nature reviews. Immunology 9: 62-70 (2009).
Roth et al., Differential expression proteomics of human colorectal cancer based on a syngeneic cellular model for the progression of adenoma to carcinoma. Proteomics 10: 194-202 (2010).
Rubinstein et al., Fusobacterium nucleatum promotes colorectal carcinogenesis by modulating E-cadherin/β-catenin signaling via its FadA adhesin. Cell Host Microbe 14: 195-206 (2013).
Sahoo et al., Extracting binary signals from microarray time-course data. Nucleic acids research 35: 3705-12 (2007).
Su et al., Increased expression of annexin A1 is correlated with K-ras mutation in colorectal cancer. The Tohoku journal of experimental medicine 222: 243-250 (2010).
Tahara et al., Fusobacterium in colonic flora and molecular features of colorectal carcinoma. Cancer research 74: 1311-1318 (2014).
Vogelstein and Kinzler, The multistep nature of cancer. Trends in genetics:TIG 9: 138-41 (1993).
Williams, Neoplastic transformation of a human colonic epithelial cell line: in vitro evidence for the adenoma to carcinoma sequence. Cancer research 50: 4724-4730 (1990).
Xu et al., FadA from Fusobacterium nucleatum utilizes both secreted and nonsecreted forms for functional oligomerization for attachment and invasion of host cells. J Biol Chem 282: 25000-25009 (2007).
Yu et al., Fusobacterium nucleatum Promotes Chemoresistance to Colorectal Cancer by Modulating Autophagy. Cell 170: 548-563 e516 (2017).
Yu et al., Invasive Fusobacterium nucleatum may play a role in the carcinogenesis of proximal colon cancer through the serrated neoplasia pathway. International journal of cancer. Journal international du cancer 139: 1318-1326 (2016).

Citation of the references herein is not intended as an admission that the reference is pertinent prior art, nor does it constitute any admission as to the contents or date of these publications or documents. In case of a conflict in terminology, the present specification controls.

The Scope of protection is defined by the appended claims.

The foregoing written specification is considered to be sufficient to enable one skilled in the art to practice the invention.

In the specification, the singular forms also include the plural forms, unless the context clearly dictates otherwise. Unless defined otherwise, all technical and scientific terms used herein have the same meaning as commonly understood by one of ordinary skill in the art to which this invention belongs. In the case of conflict, the present specification will control.

All percentages and ratios used herein, unless otherwise indicated, are by weight.

## Claims

1. An agent that inhibits or blocks Annexin A1 for use in treating or preventing cancer in a subject with familial adenomatous polyposis, wherein the agent that inhibits or blocks Annexin A1 is selected from the group consisting of an *ANXA1-*specific siRNA, an *ANXA1-* specific miRNA, DNA encoding an *ANXA1*-specific siRNA, DNA encoding an *ANXA1-* specific miRNA, and an anti-Annexin A1 antibody, minibody, Fab or fragment, camelid, or nanobody.

2. The agent for use of claim 1, wherein the cancer is selected from the group consisting of melanoma, renal cancer, prostate cancer, pancreatic adenocarcinoma, breast cancer, colon or colorectal cancer (CRC), lung cancer, esophageal cancer, squamous cell carcinoma of the head and neck, liver cancer, ovarian cancer, cervical cancer, thyroid cancer, glioblastoma, glioma, and leukemia.

3. The agent for use of claim 1, wherein the agent further comprises a vector, liposome, nanocapsule, nanoparticle, microparticle, microsphere, lipid particle, or vesicle.

4. The agent for use of claim 1, wherein the agent further comprises a pharmaceutically acceptable, diluent, carrier or adjuvant.

5. The agent for use of claim 1, wherein the agent is for use in combination with a therapeutic agent selected from the group consisting of a chemotherapeutic agent, a targeted chemotherapeutic agent, an immunotherapeutic agent and combinations thereof.

6. The agent for use of claim 5, wherein the chemotherapeutic agent is chosen from the group consisting of microtubule-targeting moietys (MTAs), DNA damaging agents, alkylating agents, antimetabolites, spindle poison plant alkaloids, cytotoxic/antitumor antibiotics, topoisomerase inhibitors, antibodies, photosensitizers, and kinase inhibitors.

7. The agent for use of claim 5, wherein the targeted chemotherapeutic agent is chosen from the group consisting of denosumab, romidepsin, ofatumumab, pazopanib, everlimus, nilotinib, temisirolimus, lapatinib, sunitinib, dasatinib, vorinostat, erlotinib, bevacizumab, cetuximab, bortezomib, gefitinib, ibritumomab, alemtuzumab, imatinib, gentuzumab, denileukin difitox, trastumab, rituximab, ramucirumab, ziv-aflibercept, panitumumab, and regorafenib.

8. The agent for use of claim 5, wherein the immunotherapeutic agent is selected from the group consisting of pembrolizumab, nivolumab, and ipilimumab.

9. The agent for use of claim 5, wherein the cancer is colorectal cancer and the chemotherapeutic agent is selected from the group consisting of 5-fluorouracil, capecitabine, irinotecan, oxaliplatin, and a combination or trifluridine and tipiracil, the targeted chemotherapeutic agent is chosen from the group consisting of bevacizumab, ramucirumab, ziv-aflibercept, cetuximab, panitumumab, and regorafenib, and the immunotherapeutic agent is selected from the group consisting of pembrolizumab, nivolumab, and ipilimumab.

10. The agent for use of claim 5, wherein the agent reduces chemo-resistance in the subject being treated for cancer with a chemotherapeutic agent.

## Patentansprüche

1. Ein Mittel, das Annexin A1 inhibiert oder blockiert, zur Verwendung bei der Behandlung oder der Vorbeugung von Krebs in einem Subjekt mit familiärer adenomatöser Polyposis, wobei das Mittel, das Annexin A1 inhibiert oder blockiert, ausgewählt ist aus der Gruppe bestehend aus einer ANXA1-spezifischen siRNA, einer ANXA1-spezifischen miRNA, DNA, die für eine ANXA1-spezifische siRNA codiert, DNA, die für eine ANXA1-spezifische miRNA codiert, und einem Anti-Annexin-A1-Antikörper, Minibody, Fab oder Fragment davon, einem Kamelid oder einem Nanobody.

2. Das Mittel zur Verwendung nach Anspruch 1, wobei der Krebs ausgewählt ist aus der Gruppe bestehend aus Melanom, Nierenkarzinom, Prostatakarzinom, Pankreas-Adenokarzinom, Brustkrebs, Darmkrebs oder kolorektalem Karzinom (CRC), Lugenkrebs, Speiseröhrenkrebs, Plattenepithelkarzinom des Kopf und Halses, Leberkrebs, Eierstockkrebs, Gebärmutterhalskrebs, Schilddrüsenkrebs, Glioblastom, Gliom und Leukämie.

3. Das Mittel zur Verwendung nach Anspruch 1, wobei das Mittel ferner einen Vektor, ein Liposom, eine Nanokapsel, ein Nanopartikel, ein Mikropartikel, eine Mikrosphäre, ein Lipidpartikel oder ein Vesikel umfasst.

4. Das Mittel zur Verwendung nach Anspruch 1, wobei das Mittel ferner ein pharmazeutisch annehmbares Verdünnungsmittel, einen pharmazeutisch annehmbaren Träger oder ein pharmazeutisch annehmbares Adjuvans umfasst.

5. Das Mittel zur Verwendung nach Anspruch 1, wobei das Mittel zur Verwendung in Kombination mit einem therapeutischen Mittel ausgewählt aus der Gruppe bestehend aus einem Chemotherapeutikum, einem zielgerichteten Chemotherapeutikum, einem Immuntherapeutikum und Kombinationen davon ist.

6. Das Mittel zur Verwendung nach Anspruch 5, wobei das Chemotherapeutikum ausgewählt ist aus der Gruppe bestehend aus gegen Mikrotubuli gerichteten Einheiten (MTAs), DNA-schädigenden Mitteln, Alkylierungsmitteln, Antimetaboliten, pflanzlichen Spindelgift-Alkaloiden, zytotoxischen/antitumoralen Antibiotika, Topoisomerase-Inhibitoren, Antikörpern, Photosensibilisatoren und Kinase-Inhibitoren.

7. Das Mittel zur Verwendung nach Anspruch 5, wobei das zielgerichtete Chemotherapeutikum ausgewählt ist aus der Gruppe bestehend aus Denosumab, Romidepsin, Ofatumumab, Pazopanib, Everolimus, Nilotinib, Temsirolimus, Lapatinib, Sunitinib, Dasatinib, Vorinostat, Erlotinib, Bevacizumab, Cetuximab, Bortezomib, Gefitinib, Ibritumomab, Alemtuzumab, Imatinib, Gemtuzumab, Denileukin diftitox, Trastuzumab, Rituximab, Ramucirumab, Ziv-Aflibercept, Panitumumab und Regorafenib.

8. Das Mittel zur Verwendung nach Anspruch 5, wobei das Immuntherapeutikum ausgewählt ist aus der Gruppe bestehend aus Pembrolizumab, Nivolumab und Ipilimumab.

9. Das Mittel zur Verwendung nach Anspruch 5, wobei der Krebs Darmkrebs ist und das Chemotherapeutikum ausgewählt ist aus der Gruppe bestehend aus 5-Fluorouracil, Capecitabin, Irinotecan, Oxaliplatin und einer Kombination aus Trifluridin und Tipiracil, das zielgerichtete Chemotherapeutikum ausgewählt ist aus der Gruppe bestehend aus Bevacizumab, Ramucirumab, Ziv-Aflibercept, Cetuximab, Panitumumab und Regorafenib, und das Immuntherapeutikum ausgewählt ist aus der Gruppe bestehend aus Pembrolizumab, Nivolumab und Ipilimumab.

10. Das Mittel zur Verwendung nach Anspruch 5, wobei das Mittel bei dem Subjekt, das mit einem Chemotherapeutikum gegen Krebs behandelt wird, die Chemoresistenz verringert.

## Revendications

1. Agent qui inhibe ou bloque l'annexine A1 pour une utilisation dans le traitement ou la prévention d'un cancer chez un sujet atteint de polypose adénomateuse familiale, sachant que l'agent qui inhibe ou bloque l'annexine A1 est sélectionné dans le groupe constitué d'un siARN spécifique d'*ANXA1*, d'un miARN spécifique d'*ANXA1*, d'un ADN codant un siARN spécifique d'*ANXA1*, d'un ADN codant un miARN spécifique d'*ANXA1*, et d'un anticorps anti-annexine A1, d'un minicorps, d'un Fab ou fragment, d'un camélidé, ou d'un nanocorps.

2. L'agent pour une utilisation de la revendication 1, sachant que le cancer est sélectionné dans le groupe constitué du mélanome, du cancer du rein, du cancer de la prostate, de l'adénocarcinome pancréatique, du cancer du sein, du cancer du côlon ou colorectal (CRC), du cancer du poumon, du cancer de l'œsophage, du carcinome épidermoïde de la tête et du cou, du cancer du foie, du cancer de l'ovaire, du cancer du col de l'utérus, du cancer de la thyroïde, du glioblastome, du gliome, et de la leucémie.

3. L'agent pour une utilisation de la revendication 1, sachant que l'agent comprend en outre un vecteur, un liposome, une nanocapsule, une nanoparticule, une microparticule, une microsphère, une particule lipidique, ou une vésicule.

4. L'agent pour une utilisation de la revendication 1, sachant que l'agent comprend en outre un diluant, un support ou un adjuvant pharmaceutiquement acceptable.

5. L'agent pour une utilisation de la revendication 1, sachant que l'agent est destiné à être utilisé en combinaison avec un agent thérapeutique sélectionné dans le groupe constitué d'un agent chimiothérapeutique, d'un agent chimiothérapeutique ciblé, d'un agent immunothérapeutique et de combinaisons de ceux-ci.

6. L'agent pour une utilisation de la revendication 5, sachant que l'agent chimiothérapeutique est choisi dans le groupe constitué d'entités ciblant les microtubules (MTA), d'agents endommageant l'ADN, d'agents alkylants, d'antimétabolites, d'alcaloïdes végétaux poisons du fuseau mitotique, d'antibiotiques cytotoxiques/antitumoraux, d'inhibiteurs de topoisomérase, d'anticorps, de photosensibilisateurs, et d'inhibiteurs de kinase.

7. L'agent pour une utilisation de la revendication 5, sachant que l'agent chimiothérapeutique ciblé est choisi dans le groupe constitué du dénosumab, de la romidepsine, de l'ofatumumab, du pazopanib, de l'évérolimus, du nilotinib, du temisirolimus, du lapatinib, du sunitinib, du dasatinib, du vorinostat, de l'erlotinib, du bévacizumab, du cétuximab, du bortézomib, du géfitinib, de l'ibritumomab, de l'alemtuzumab, de l'imatinib, du gentuzumab, de la dénileukine difitox, du trastumab, du rituximab, du ramucirumab, du ziv-aflibercept, du panitumumab, et du régorafénib.

8. L'agent pour une utilisation de la revendication 5, sachant que l'agent immunothérapeutique est sélectionné dans le groupe constitué du pembrolizumab, du nivolumab, et de l'ipilimumab.

9. L'agent pour une utilisation de la revendication 5, sachant que le cancer est un cancer colorectal et l'agent chimiothérapeutique est sélectionné dans le groupe constitué du 5-fluoro-uracile, de la capécitabine, de l'irinotécan, de l'oxaliplatine, et d'une combinaison de trifluridine et de tipiracil, l'agent chimiothérapeutique ciblé est choisi dans le groupe constitué du bévacizumab, du ramucirumab, du ziv-aflibercept, du cétuximab, du panitumumab, et du régorafénib, et l'agent immunothérapeutique est sélectionné dans le groupe constitué du pembrolizumab, du nivolumab, et de l'ipilimumab.

10. L'agent pour une utilisation de la revendication 5, sachant que l'agent réduit la chimiorésistance chez le sujet qui est traité pour un cancer avec un agent chimiothérapeutique.
